(19) Europäisches Patentamt
European Patent Office
Office européen des brevets

(11) Veröffentlichungsnummer: **0 148 119 B1**

(12) **EUROPÄISCHE PATENTSCHRIFT**

(45) Veröffentlichungstag der Patentschrift: **03.05.89**

(21) Anmeldenummer: **84810586.2**

(22) Anmeldetag: **30.11.84**

(51) Int. Cl.[4]: **C 07 C 125/08**, C 07 D 213/64, C 07 D 215/22, C 07 D 241/44, C 07 D 263/58, C 07 D 277/68, A 01 N 43/40, A 01 N 43/42, A 01 N 43/76, A 01 N 43/78

# (54) 2-Phenoxypropionsure-cyanamide.

**Teilanmeldung 87117526 eingereicht am 27.11.87.**

(30) Priorität: **06.12.83 CH 6509/83**
**18.04.84 CH 1948/84**

(43) Veröffentlichungstag der Anmeldung:
**10.07.85 Patentblatt 85/28**

(45) Bekanntmachung des Hinweises auf die Patenterteilung:
**03.05.89 Patentblatt 89/18**

(84) Benannte Vertragsstaaten:
**AT BE CH DE FR GB IT LI NL SE**

(56) Entgegenhaltungen:
**EP-A- 0 021 453**
**DE-A- 2 640 730**
**DE-A- 3 004 770**

(73) Patentinhaber: **CIBA-GEIGY AG, Klybeckstrasse 141, CH-4002 Basel (CH)**

(72) Erfinder: **Böhner, Beat, Dr., Hügelweg 3, CH-4102 Binningen (CH)**
Erfinder: **Rempfler, Hermann, Dr., Brücklismattstrasse 16, CH-4107 Ettingen (CH)**
Erfinder: **Schurter, Rolf, Dr., Holzmattstrasse 45, CH-4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 148 119 B1

**Beschreibung**

Die vorliegende Erfindung betrifft neue herbizid wirksame 2-Phenoxy-propionsäure-cyanamide, Verfahren zu ihrer Herstellung, ferner herbizide Mittel, die diese neuen Verbindungen als Wirkstoffe enthalten, sowie die Verwendung der neuen Wirkstoffe und der sie enthaltenden Mittel zur selektiven Bekämpfung von Unkräutern in Kulturpflanzenbeständen. Ferner betrifft die Erfindung auch neue Zwischenprodukte für die Synthese der erfindungsgemässen Verbindungen.

Die erfindungsgemässen 2-Phenoxypropionsäure-cyanamide entsprechen der Formel I

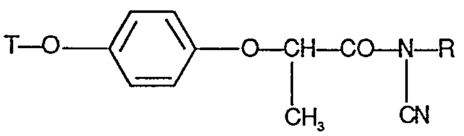 (I)

worin
R Wasserstoff, $C_1$–$C_4$-Alkyl, $C_3$–$C_4$-Alkenyl, $C_3$–$C_4$-Alkinyl oder $C_2$–$C_4$-Alkoxyalkyl bedeutet und
T für einen Rest

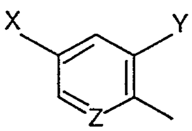 ,

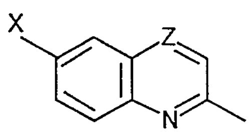 oder 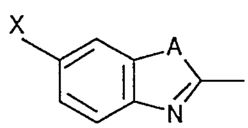

steht, in welchen
A Sauerstoff oder Schwefel,
X Fluor, Chlor, Brom, Jod oder Trifluormethyl,
Y Wasserstoff, Fluor, Chlor, Brom oder Trifluormethyl und
Z Stickstoff oder die Methinbrücke bedeuten.

Herbizide 2-Phenoxypropionsäure-Amide mit weiteren Substituenten in der para-Stellung des Phenylkerns sind aus der Literatur, wie beispielsweise den Deutschen Offenlegungsschriften 2 433 067, 2 531 643, 2 639 796, 2 640 730 oder 3 004 770 oder der Europäischen Patentanmeldung EP-A 21 453 bekannt.

Es wurde nun überraschenderweise gefunden, dass die neuen Wirkstoffe gemäss vorliegender Erfindung den literaturbeschriebenen Verbindungen dieser Substanzklasse in der selektiven Unkrautbekämpfung in Nutzpflanzenkulturen überlegen sind.

Im Rahmen der vorliegenden Erfindung fallen unter die in der Definition der Formel I verwendeten Symbole R und T beispielsweise folgende Substituenten: R steht im allgemeinen für Wasserstoff, Methyl, Äthyl, Isopropyl, n-Propyl, die vier isomeren Butyl, Allyl, Methallyl, 2-Butenyl, 3-Butenyl, Propargyl, 2-Butinyl, 3-Butinyl, Methoxymethyl, Methoxyäthyl, Äthoxymethyl sowie Äthoxyäthyl; und T steht im allgemeinen für einen mindestens durch das Radikal X substituierten Rest aus der Reihe Phenyl, 2-Pyridinyl, 2-Chinolinyl, 2-Chinoxalinyl, 2-Benzoxazolyl oder 2-Benzthiazolyl. Vorzugsweise bedeutet R Wasserstoff oder einen gesättigten Rest wie $C_1$–$C_4$-Alkyl oder $C_2$–$C_4$-Alkoxyalkyl und T einen Rest wie 2-Benzoxazolyl oder 2-Chinoxalinyl.

Wegen ihrer guten selektivherbiziden Wirkung sind unter den Verbindungen der Formel I diejenigen hervorzuheben, in denen entweder
a) R für Wasserstoff oder $C_1$–$C_4$-Alkyl steht oder

b) T für den Rest 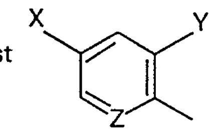 steht oder

c) T für den Rest 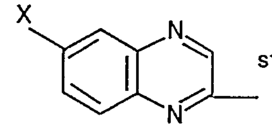 steht oder

d) T für den Rest 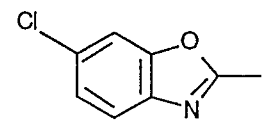 steht.

Innerhalb der Untergruppe a) sind die Verbindungen bevorzugt, in denen R für $C_1$–$C_4$-Alkyl steht; in der Untergruppe b) diejenigen, in denen entweder X für Trifluormethyl, Y für Wasserstoff und Z für die Methinbrücke; oder X für Trifluormethyl, Y für Wasserstoff und Z für Stickstoff; oder X und Y für Chlor und Z für Stickstoff; oder X für Chlor, Y für Fluor und Z für Stickstoff stehen; d.h. T einen Rest aus der Gruppe 4-Trifluormethylphenyl, 5-Trifluormethylpyridin-2-yl, 3,5-Dichlorpyridin-2-yl und 5-Chlor-3-fluorpyridin-2-yl repräsentiert.

Innerhalb der Gruppe c) sind diejenigen bevorzugt, in denen X für Fluor oder Chlor steht.

Ganz besonders bevorzugten Untergruppen von Verbindungen der Formel I sind dadurch charakterisiert, dass R für $C_1$–$C_4$-Alkyl und T entweder für 4-Trifluormethylphenyl oder 5-Trifluormethylpyridin-2-yl oder 3,5-Dichlorpyridin-2-yl oder 5-Chlor-3-fluorpyridin-2-yl stehen, wobei der letzte Typ Vorrang geniesst; oder dass R für $C_1$–$C_4$-Alkyl und T für 6-Fluor-chinoxalin-2-yl oder 6-Chlor-chinoxalin-2-yl stehen; oder dass R für $C_1$–$C_4$-Alkyl und T für 6-Chlor-benzoxazol-2-yl stehen.

Als bevorzugte Einzelverbindungen sind zu nennen:

2-[4-(5-Chlor-3-fluorpyridin-2-yloxy)-phenoxy]-propionsäure-N-cyan-N-methyl-amid,
2-[4-(5-Trifluormethylpyridin-2-yloxy)-phenoxy]-propionsäure-N-cyan-N-methyl-amid,
2-[4-(5-Chlor-3-fluorpyridin-2-yloxy)-phenoxy]-propionsäure-N-cyan-N-äthyl-amid,
2-[4-(6-Chlor-chinoxalin-2-yloxy)-phenoxy]-propionsäure-N-cyan-N-äthyl-amid,

2-[4-(6-Chlor-chinoxalin-2-yloxy)-phenoxy]-propionsäure-N-cyan-N-methyl-amid,
2-[4-(6-Fluor-chinoxalin-2-yloxy)-phenoxy]-phenoxy]-propionsäure-N-cyan-N-äthyl-amid,
2-[4-(6-Fluor-chinoxalin-2-yloxy)-phenoxy]-propionsäure-N-cyan-N-methyl-amid oder
2-[4-(6-Chlor-benzoxazol-2-yloxy)-phenoxy]-propionsäure-N-cyan-N-n-butyl-amid.

Die erfindungsgemässen Verbindungen der Formel I können hergestellt werden, indem man ein 2-Phenoxypropionsäurehalogenid der Formel II

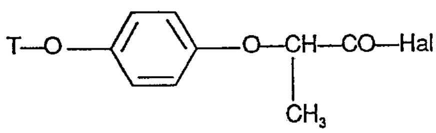 (II),

worin T die unter Formel I gegebene Bedeutung hat und Hal für Chlor oder Brom steht, in Gegenwart eines säurebindenden Mittels mit einem Cyanamin der Formel III

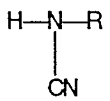 (III),

worin R die unter Formel I gegebene Bedeutung hat, umsetzt.

Für den Fall, dass R eine andere Bedeutung als Wasserstoff hat, können die Verbindungen der Unterformel Ia

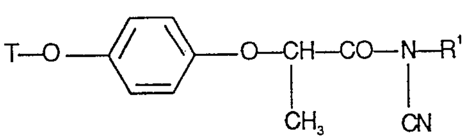 (Ia),

worin T die unter Formel I gegebene Bedeutung hat und $R^1$ für $C_1$-$C_4$-Alkyl, $C_3$-$C_4$-Alkenyl, $C_3$-$C_4$-Alkinyl oder $C_2$-$C_4$-Alkoxyalkyl steht, erhalten werden, indem man eine entsprechende Cyanamino-Verbindung der Unterformel Ib

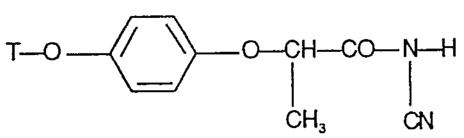 (Ib),

worin T die unter Formel I gegebene Bedeutung hat, in Gegenwart eines säurebindenden Mittels mit einer Halogenverbindung der Formel IV

Hal-$R^1$ (IV),

worin $R^1$ die unter Formel Ia gegebene Bedeutung hat und Hal für Chlor, Brom oder Jod steht, umsetzt.

Nach einem zweiten Verfahren kann man die erfindungsgemässen Verbindungen der Formel I auch erhalten, indem man ein Phenoxypropionsäurederivat der Formel V

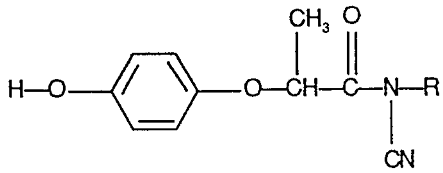 (V)

worin R die unter Formel I gegebene Bedeutung hat, in Gegenwart eines säurebindenden Mittels mit einem Halogenid der Formel VI

T-Hal (VI)

worin T die unter Formel I gegebene Bedeutung hat und Hal für Fluor, Chlor, Brom oder Jod steht, umsetzt.

Nach einem dritten Verfahren erhält man die erfindungsgemässen Verbindungen der Formel I, indem man ein Propionsäurederivat der Formel VII

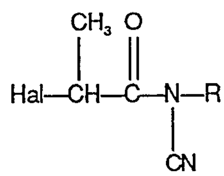 (VII)

worin R die unter Formel I gegebene Bedeutung hat und Hal für Chlor, Brom, Tosyl oder Mesyl steht, in Gegenwart eines säurebindenden Mittels mit einem Hydrochinonderivat der Formel VIII

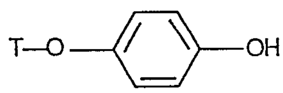 (VIII)

worin T die unter Formel I gegebene Bedeutung hat, umsetzt.

Die verschiedenen Umsetzungen zu Verbindungen der Formel I bzw. Ib → Ia, werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Äther wie Diäthyläther, Äthylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen −20° und +120°C. Als Basen sind insbesondere tertiäre Amine Trimethylamin, Triäthylamin, Chinuclidin, 1,4-Diazabicyclo [2.2.2]octan, 1,5-Diazabicyclo-[4.3.0]non-5-en oder 1,8-Diazabicyclo[5.4.0]-undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Zwischenprodukte der Formeln II, III, IV, VI

und VIII sind grösstenteils bekannt oder können analog zu bekannten Verfahren hergestellt werden.

Die Zwischenprodukte der Formeln V und VIII sind neu und wurden speziell für die Synthese der Verbindungen der Formel I entwickelt und hergestellt. Sie bilden daher weitere Aspekte der vorliegenden Erfindung.

Das Propionsäurederivat der Formel VII

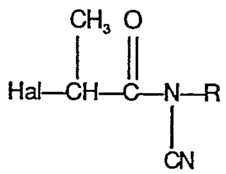

(VII)

wird erhalten, indem man ein Propionsäurehalogenid der Formel IX

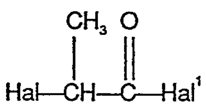

(IX)

worin Hal die unter Formel VII gegebene Bedeutung hat und $Hal^1$ für Chlor oder Brom steht, in Gegenwart eines säurebindenden Mittels mit einem Cyanamin der Formel X

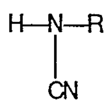

(X)

worin R die unter Formel VII gegebene Bedeutung hat, umsetzt.

Nach einer Variante des obigen Verfahrens, kann man auch zunächst die Verbindungen der Unterformel VIIa

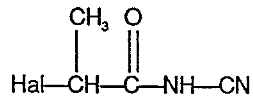

(VIIa)

herstellen, indem man das Propionsäurehalogenid der Formel IX zunächst in Gegenwart eines säurebindenden Mittels mit Cyanamin der Formel XI

$H_2N$-CN (XI)

umsetzt, und diese Verbindungen der Formel VIIa gewünschtenfalls mit einem Alkylierungsreagenz, das den Rest $R^1$, wie unter Formel IV definiert, überträgt, umsetzt.

Die Zwischenprodukte der Formel V können aus den Verbindungen der Formel VII hergestellt werden, indem man die Verbindung der Formel VII

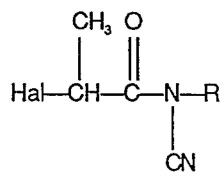

(VII)

in Gegenwart einer Base mit 4-Benzyloxyphenol umsetzt und das erhaltene Produkt der Formel XII

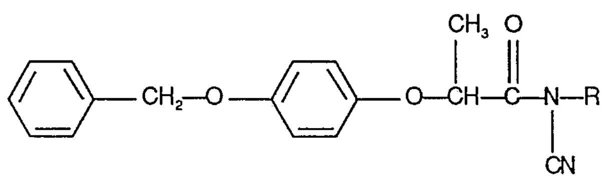

(XII)

worin R die unter Formel I gegebene Bedeutung hat in Gegenwart eines Hydrierungskatalysators, wie beispielsweise Palladium/Kohle, mit Wasserstoff behandelt.

Die Ausgangsmaterialien der Formeln IX, X oder XI sind bekannt oder können nach bekannten Methoden hergestellt werden. Die neuen Zwischenprodukte der Formel XII wurden speziell für die Synthese der Verbindungen der Formel I entworfen und hergestellt. Sie bilden daher einen weiteren Erfindungsgegenstand.

Die Herstellung der neuen Zwischenprodukte der Formeln V und VII erfolgt nach an sich bekannten Methoden. Die Reaktionsbedingungen werden jeweils nach den Erfordernissen der eingesetzten Reagenzien gewählt.

Über ein neues Verfahren kann man Verbindungen der Unterformel Ic

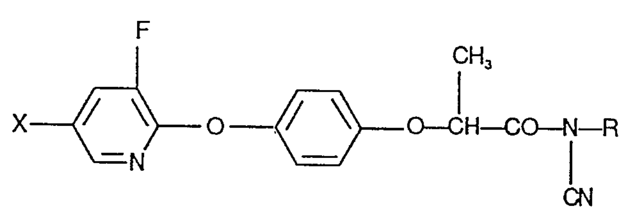

(Ic)

worin R und X die unter Formel I gegebene Bedeutung haben herstellen, indem man entweder

a) ein 3-Nitropyridin der Formel XIII

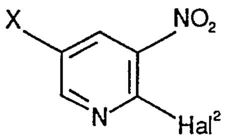

(XIII)

worin X die unter Formel I gegebene Bedeutung hat und $Hal^2$ für Fluor, Chlor oder Brom steht, in Gegenwart einer Base mit Hydrochinon zu einer Verbindung der Formel XIV

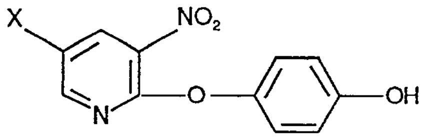

(XIV)

umsetzt, dieses Zwischenprodukt in Gegenwart eines Metallkatalysators zu einer Aminoverbindung der Formel XV

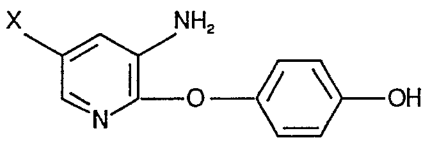

(XV)

reduziert, diese diazotiert und mit einem Fluorierungsmittel in eine Verbindung der Formel XVI

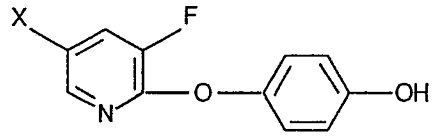

(XVI)

überführt und dieses fluorierte Zwischenprodukt in Gegenwart einer Base mit einem Propionsäure-Derivat der Formel VII umsetzt, oder indem man

b) das Nitropyridin der Formel (XIII) in Gegenwart einer Base mit einem Propionsäureester der Formel XVII

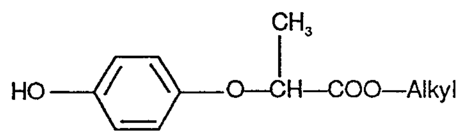

(XVII)

worin Alkyl für $C_1$-$C_4$-Alkyl steht, umsetzt, das erhaltene Zwischenprodukt der Formel XVIII

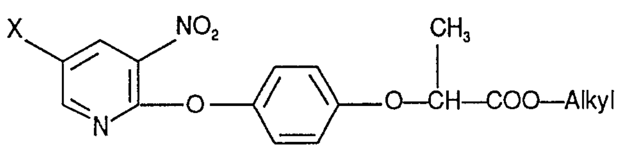

(XVIII)

in Gegenwart eines Metallkatalysators zur Aminoverbindung der Formel XIX

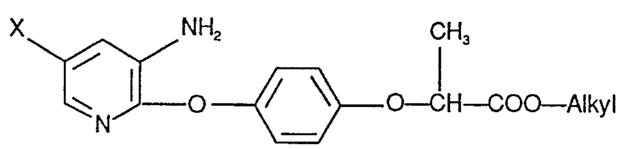

(XIX)

reduziert, dieses Zwischenprodukt diazotiert und mit einem Fluorierungsmittel in eine Verbindung der Formel XX

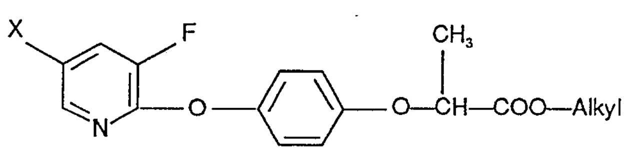

(XX)

überführt, diesen Ester verseift und mit einem Halogenierungsmittel in ein 2-Phenoxypropionsäurehalogenid der Unterformel IIa

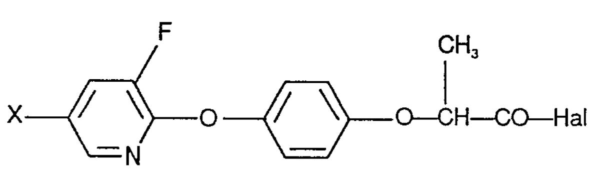

(IIa)

worin Hal und X die unter Formel II gegebene Bedeutung haben, überführt und in Gegenwart einer Base mit einem Cyanamin der Formel III umsetzt.

Gemäss einer Variante des obigen Verfahrens kann man die Zwischenprodukte der Formel XVIII auch erhalten, indem man die Verbindungen der Formel XIV in Gegenwart einer Base mit einem Propionsäureester der Formel XXI

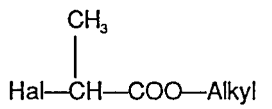

(XXI)

worin Hal für Chlor oder Brom und Alkyl für $C_1$-$C_4$-Alkyl stehen, umsetzt.

Die Reaktionsbedingungen für die an sich bekannten Reaktionsschritte, wie Katalysatoren, Lösungsmittel und Reaktionstemperaturen, sind aus der Literatur bekannt.

Die Ausgangsprodukte der Formeln XIII und XVII sind bekannt.

Die durchlaufenen Zwischenprodukte der Formeln XIV, XV und XVIII sind neu und wurden speziell für die Synthese der Verbindungen der Formel Ic entwickelt. Das neue Verfahren mit den Varianten a und b bildet einen Gegenstand der vorliegenden Erfindung. Ebenfalls neu sind die Verbindungen der Formel IIa, worin X für Halogen steht.

Die Verbindungen der Formel I fallen als Racemate an. Gegenstand der Erfindung sind beide Enantiomeren der Wirkstoffe der Formel I und Gemische davon. Sofern nicht ausdrücklich ein bestimmtes Isomeres erwähnt ist, beziehen sich die in der Beschreibung gemachten Feststellungen stets auf die Racemate.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektiv-herbizide Eigenschaften, insbesondere gegen monokotyle Unkräuter aus, die sie ausgezeichnet zum Einsatz in Kulturen von dikotylen Nutzpflanzen, insbesondere Baumwolle, Soja, Raps, Zukker- und Futterrüben sowie Sonnenblumen, befähigen. Zum Teil können die erfindungsgemässen Verbindungen der Formel I aber auch als Selektivherbizide in monokotylen Kulturen wie Getreide, beispielsweise Weizen und Gerste, eingesetzt werden. Es werden dabei teilweise auch Unkräuter geschädigt, welchen bisher nur mit Totalherbiziden beizukommen war.

Bei höheren Aufwandmengen entfalten die Wirkstoffe der Formel I totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung, insbesondere gegen Schadgräser, gleichermassen mit gutem Erfolg verwendet werden.

Die Erfindung betrifft auch herbizide Mittel, welche einen neuen Wirkstoff der Formel I enthalten, sowie Verfahren zur pre- und postemergenten Unkrautbekämpfung von monokotylen Pflanzen, insbesondere Gräsern.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Öl- oder Stearinsäure, oder von natürlichen Fettsäuregemischen, die z.B. aus Kokosnuss- oder Talgöl gewonnen werden können, genannt. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäuresters oder eines aus natürlichen Fettsäuren hergestellten Fettalkoholsulfatgemisches.

Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing corp., Ridgewood, New Jersey, 1981;
H. Stache, «Tensid-Taschenbuch», 2. Aufl., C. Hanser Verlag, München, Wien, 1981;
M. and J. Ash. «Encyclopedia of Surfactants», Vol. I-III, Chemical Publishing Co., New York, 1980-1981.

Die pestiziden Zubereitungen enthalten in der Regel 0,1 bis 95%, insbesondere 0,1 bis 80% Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent).

Emulgierbare Konzentrate
Aktiver Wirkstoff:
1 bis 20%, bevorzugt 5 bis 10%
oberflächenaktive Mittel:
5 bis 30%, vorzugsweise 10 bis 20%
flüssige Trägermittel:
50 bis 94%, vorzugsweise 70 bis 85%

Stäube
Aktiver Wirkstoff:
0,1 bis 10%, vorzugsweise 0,1 bis 1%
festes Trägermittel:
99,9 bis 90%, vorzugsweise 99,9 bis 99%

Suspensions-Konzentrate
Aktiver Wirkstoff:
5 bis 75%, vorzugsweise 10 bis 50%
Wasser:
94 bis 25%, vorzugsweise 90 bis 30%
oberflächenaktives Mittel:
1 bis 40%, vorzugsweise 2 bis 30%

Benetzbares Pulver
Aktiver Wirkstoff:
0,5 bis 90%, vorzugsweise 1 bis 80%
oberflächenaktives Mittel:
0,5 bis 20%, vorzugsweise 1 bis 15%
festes Trägermittel:
5 bis 95%, vorzugsweise 15 bis 90%

Granulate
Aktiver Wirkstoff:
0,5 bis 30%, vorzugsweise 3 bis 15%
festes Trägermittel:
99,5 bis 70%, vorzugsweise 97 bis 85%.

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Beispiel H1

2-[4-(4-Trifluormethylphenoxy)-phenoxy]-propionsäure-N-cyanamid (Verb. Nr. 1.4).

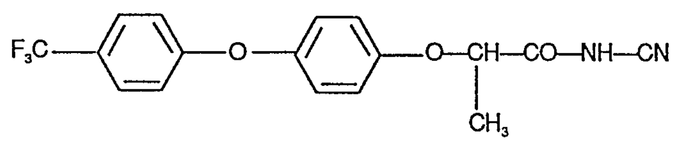

10,4 g (0,1575 Mol) 85%iges Kaliumhydroxid werden in 75 ml Wasser gelöst. Bei einer Temperatur von 20°C setzt man 3,5 g (0,0825 Mol) Cyanamid zu und kühlt die klare, farblose Lösung auf 10°C ab. Anschliessend wird eine Lösung von 25,8 g (0,075 Mol) 2-[4-(4-Trifluormethylphenoxy)-phenoxy]-propionsäurechlorid in 15 ml Aceton so zugetropft, dass die Temperatur 15°C nicht übersteigt. Nachdem die Reaktionsmischung für 45 Minuten bei dieser Temperatur gerührt worden ist, lässt man 9 g (0,09 Mol) 37%ige wässrige Salzsäure bei 5°C zutropfen. Dabei fällt das Produkt ölig aus, kristallisiert jedoch beim Stehen innerhalb von 30 Minuten. Die farblosen Kristalle werden abfiltriert, mit Wasser gewaschen. Man erhält nach dem Trocknen über Phosphorpentoxid so 24,9 g (95,0% d. Th.) 2-[4-(4-Trifluormethylphenoxy)-phenoxy]-propionsäure-N-cyanamid, Smp. 125–127°C

Beispiel H2

2-[4-(4-Trifluormethylphenoxy)-phenoxy]-propionsäure-N-cyan-N-methylamid (Verb. Nr. 1.3).

17,5 g (0,05 Mol) 2-[4-(4-Trifluormethylphenoxy)-phenoxy]-propionsäure-N-cyanamid werden in 60 ml Methyläthylketon gelöst und zusammen mit 7,6 g (0,055 Mol) Kaliumcarbonat für 30 Minuten zum Rückfluss erhitzt. Zu der farblosen Suspension setzt man dann bei einer Temperatur von 30°C 3,6 ml (0,057 Mol) Methyljodid zu und rührt die Mischung für weitere 2 Stunden bei der gleichen Temperatur. Das Reaktionsgemisch wird nun filtriert und das Filtrat eingedampft. Den öligen Rückstand chromatographiert man mit Petroläther/Essigester (3:1) an Kieselgel und erhält beim Eindampfen der Hauptfraktion 10,9 g (59,9% d. Th.) 2-[4-(4-Trifluormethylphenoxy)-phenoxy]-propionsäure-N-cyan-N-methylamid als farbloses klares Öl mit einem Brechungsindex von $n_D^{23}$ = 1,5228.

Beispiel H3

2-[4-(6-Chlorbenzoxazol-2-yloxy)-phenoxy]-propionsäure-N-cyan-N-butylamid (Verb. Nr. 2.4).

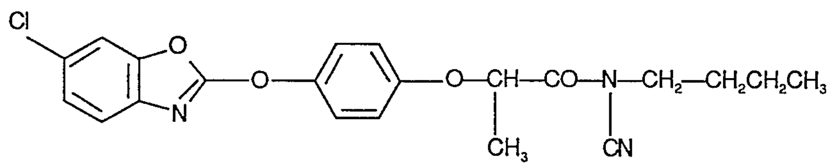

a) 2-Brompropionsäure-N-cyan-N-methylamid.

10,5 g (0,25 Mol) Cyanamid werden in 250 ml 2N Kaliumhydroxidlösung gelöst und auf +10°C abgekühlt. Bei dieser Temperatur lässt man 26,5 ml (0,25 Mol) 2-Brompropionsäurebromid zutropfen und rührt das Reaktionsgemisch für eine Stunde bei gleicher Temperatur. Dann setzt man 16,8 g (0,2 Mol) Natriumhydrogencarbonat zu. Bei einer Temperatur von 20°C lässt man 63 g (0,5 Mol) Dimethylsulfat zutropfen. Nach Rühren für 4 Stunden wird das Reaktionsgemisch mit Methylenchlorid extrahiert, die vereinigten organischen Phasen werden mit Natriumsulfat getrocknet und eingedampft. Als Rückstand verbleiben 27,5 g Rohprodukt. Durch Vakuumdestillation erhält man daraus 13,6 g (28,5% der Theorie) 2-Brompropionsäure-N-cyan-N-methylamid, Sdp. 45-47°C bei 0,008 mbar.

b) 2-(4-Benzyloxyphenoxy)-propionsäure-N-cyan-N-methylamid.

Zu einer Lösung von 20,0 g (0,1 Mol) Hydrochinonmonobenzyläther und 16,5 g (0,12 Mol) Kaliumcarbonat in 200 ml Dimethylformamid werden unter starkem Rühren 19,1 g (0,1 Mol) 2-Brompropionsäure-N-cyan-N-methylamid tropfenweise zugesetzt. Nachdem die leicht exotherme Reaktion abgeklungen ist, wird das Reaktionsgemisch für 3 Std. auf 40°C erhitzt. Dann werden die ausgefallenen Salze abfiltriert und das Filtrat eingedämpft. Der Rückstand wird in Äther gelöst und mit verdünnter Natriumhydroxidlösung ausgeschüttelt. Nach dem Trocknen der organischen

Phase mit Natriumsulfat wird das Lösungsmittel eingedampft und der Rückstand im Vakuum destilliert. Man erhält 19,5 g (63% der Theorie) 2-(4-Benzyloxyphenoxy)-propionsäure-N-cyan-N-methylamid, Smp. 56–58 °C.

c) 2-(4-Hydroxyphenoxy)-propionsäure-N-cyan-N-butylamid.

16,5 g (0,047 Mol) 2-(4-Benzyloxyphenoxy)-propionsäure-N-cyan-N-butylamid, hergestellt analog Beispiel b), werden in 170 ml Dioxan in Gegenwart von 1,7 g Palladium/Kohle-Katalysator (5%ig) mit Wasserstoff hydriert. Anschliessend wird der Katalysator abfiltriert und das Filtrat eingedampft. Durch Chromatographie mit Äthylacetat/Hexan (1:3) an Kieselgel erhält man 7,5 g (61% d. Th.)

2-(4-Hydroxyphenoxy)-propionsäure-N-cyan-butylamid als farbloses

Oel, $n_D^{25}$: 1,5132.

d) 4,4 g (0,017 Mol) 2-(Hydroxyphenoxy)-propionsäure-N-cyan-N-butylamid, 3,8 g (0,02 Mol) 2,6-Dichlorbenzoxazol und 3,5 g (0,025 Mol) Kaliumcarbonat werden in 50 ml Acetonitril für 10 Std. bei Raumtemperatur gerührt. Anschliessend werden die Salze abfiltriert und das Filtrat eingedampft. Durch Chromatographie an Kieselgel mit Äthylenacetat/Hexan (1:3) erhält man 2-[4-(6-Chlorbenzoxazol-2-yloxy)-phenoxy]-propionsäure-N-cyan-N-butylamid, Smp. 91–92 °C.

Beispiel H4

2-Brompropionsäure-N-cyan-N-butylamid.

Zu 13,4 g (0,137 Mol) Butylcyanamid und 13,8 g (0,137 Mol) Triäthylamin in 150 ml Äther werden bei einer Temperatur von 15 °C 29,5 g (0,137 Mol) 2-Brompropionsäurebromid tropfenweise zugesetzt. Nach Rühren für 3 Std. bei einer Temperatur von 20° bis 25 °C wird das gebildete Salz abfiltriert, das Filtrat mit verdünnter Salzsäure ausgeschüttelt, mit Natriumsulfat getrocknet und eingedampft. Der ölige Rückstand wird im Hochvakuum destilliert. Man erhält 22,6 g (70,8% der Theorie) 2-Brompropionsäure-N-cyan-N-butylamid, Sdp. 53–54 °C bei 0,0052 mbar.

Beispiel H5

2-[4-(5-Chlor-3-fluorpyridin-2-yloxy)-phenoxy]-propionsäure-N-cyan-N-methylamid (Verb. Nr. 1.26).

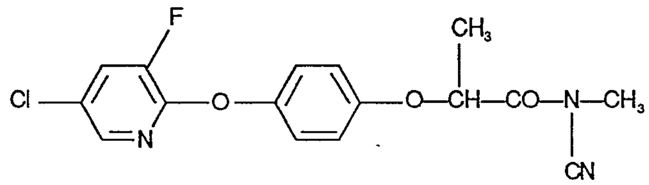

a) 4-(5-Chlor-3-nitropyridin-2-yloxy)-phenol.

Ein Gemisch aus 85,9 g (0,78 Mol) Hydrochinon, 1200 ml Acetonitril und 53,9 g (0,39 Mol) Kaliumcarbonat wird auf eine Temperatur von 60°C erwärmt. Dann lässt man innerhalb von 6 Stunden eine Lösung von 115,8 g (0,60 Mol) 2,5-Dichlor-3-nitro-pyridin in 500 ml Acetonitril zutropfen und rührt die Mischung für weitere 36 Stunden. Das Lösungsmittel wird abdestilliert und der Rückstand in ein Eiswasser/Salzsäure-Gemisch gegossen. Nach der Extraktion mit Methylenchlorid werden die vereinigten organischen Phasen über Magnesiumsulfat getrocknet, mit Aktivkohle behandelt, filtriert und eingeengt. Der Rückstand wird durch Kristallisation in Hexan/Äthylacetat-Gemisch gereinigt. Man erhält 99 g (62% d. Th.) 4-(5-Chlor-3-nitropyridin-2-yloxy)-phenol, Smp. 125–126 °C.

b) 4-(3-Amino-5-chlorpyridin-2-yloxy)-phenol.

110,7 g (0,415 Mol) 4-(5-Chlor-3-nitropyridin-2-yloxy)-phenol werden in 1200 ml Dioxan gelöst und, nach Zugabe von 22,0 g Raney-Nickel-Katalysator, bei 20–25 °C mit Wasserstoff hydriert. Dann wird der Katalysator abfiltriert, die Lösung eingeengt und der Rückstand mit Hexan verrührt. Nach dem Filtrieren und Trocknen werden 96,0 g (98% d. Th.) 4-(3-Amino-5-chlorpyridin-2-yloxy)-phenol erhalten, Smp. 174 °C.

c) 4-(5-Chlor-3-fluorpyridin-2-yloxy)-phenol.

In 400 g (20 Mol) vorgelegten Fluorwasserstoff trägt man bei einer Temperatur zwischen −8 °C und 0 °C 122,5 g (0,518 Mol) 4-(3-Amino-5-chlorpyridin-2-yloxy)-phenol ein. Dann werden innerhalb von einer Stunde portionsweise 37,3 g (0,540 Mol) Natriumnitrit eingetragen. Das Gemisch wird für 2 Stunden bei 0 °C gerührt und im Autoklaven langsam auf 55 °C erwärmt. Das überflüssige Fluorwasserstoff wird abdestilliert, der Rückstand mit 200 ml Methylenchlorid aufgenommen, mit Eiswasser und Ammoniak neutralisiert. Nach Trocknen und Eindampfen der organischen Phase erhält man 112 g 4-(5-Chlor-3-fluorpyridin-2-yloxy)-phenol, Smp. 97–98 °C.

d) 48,0 g (0,20 Mol) 4-(5-Chlor-3-fluorpyridin-2-yloxy)-phenol und 38,2 g (0,20 Mol) 2-Brompropionsäure-N-cyan-N-methylamid werden in 400 ml Acetonitril gelöst, mit 35,9 g (0,26 Mol) Kaliumcarbonat und 0,33 g (0,002 Mol) Kaliumjodid versetzt und für 13 Stunden auf 80 °C erhitzt. Der Niederschlag wird abgetrennt, das Filtrat eingedampft und der Rückstand in 500 ml Methylenchlorid aufgenommen. Zur Reinigung wird die Lösung mit Aktivkohle behandelt und durch Kieselgel filtriert. Das Filtrat wird eingedampft und der Rückstand aus Hexan/Äthylacetat-Gemisch kristallisiert, wodurch man 61 g 2-[4-(5-Chlor-3-fluorpyridin-2-yloxy)-phenoxy]-propionsäure-N-cyan-N-methylester erhält.

Beispiel H6

2-[4-(5-Chlor-3-fluorpyridin-2-yloxy)-phenoxy]-propionsäure-N-n-butyl-N-cyanamid (Verb. Nr. 1.28).

a) 2-[4-(5-Chlor-3-nitropyridin-2-yloxy)-phenoxy]-propionsäuremethylester.

38,6 g (0,20 Mol) 2,5-Dichlor-3-nitropyridin, 41,2 g (0,21 Mol) 2-(4-Hydroxyphenoxy)-propionsäuremethylester, 400 ml Acetonitril, 35,9 g (0,26 Mol) Kaliumcarbonat und 0,33 g (0,002 Mol) Kaliumjodid werden für 13 Stunden auf eine Temperatur von 80 °C erhitzt. Das Reaktionsgemisch wird filtriert und der Filterkuchen mit Acetonitril nachgewaschen. Das Filtrat wird eingedampft und der Rückstand in 500 ml Methylenchlorid aufgenommen. Nach Behandlung der Lösung mit 15 g Aktivkohle wird sie durch eine Schicht Kieselgel filtriert und das Filtrat eingedampft. Der Rückstand wird aus Hexan/Äthylacetat-Gemisch kristallisiert. Man erhält 65,7 g (93,1% d. Th.) 2-[4-(5-Chlor-3-nitropyridin-2-yloxy)-phenoxy]-propionsäuremethylester, Smp. 91–92 °C.

b) 2-[4-(3-Amino-5-chlorpyridin-2-yloxy)-phenoxy]-propionsäuremethylester.

190,8 g (0,541 Mol) 2-[4-(5-Chlor-3-nitropyridin-2-yloxy)-phenoxy]-propionsäuremethylester werden in 1,9 l Dioxan gelöst, mit 40 g Raney-Nickel-Katalysator versetzt und bei 20–25 °C mit Wasserstoff hydriert. Anschliessend wird der Katalysator abfiltriert, das Lösungsmittel abdestilliert und der noch warme ölige Rückstand unter Einrühren in Hexan kristallisiert. Der Niederschlag wird abgetrennt und getrocknet. Man erhält so 167,4 g (96% d. Th.) 2-[4-(3-Amino-5-chlorpyridin-2-yloxy)-phenoxy]-propionsäuremethylester, Smp. 92–94 °C.

c) 2-[4-(5-Chlor-3-fluorpyridin-2-yloxy)-phenoxy]-propionsäuremethylester.

400 g (20 Mol) Fluorwasserstoff werden vorgelegt und bei einer Temperatur zwischen −8 °C und 0 °C portionsweise mit 167,4 g (0,518 Mol) 2-[4-(3-Amino-5-chlorpyridin-2-yloxy)-phenoxy]-propionsäuremethylester versetzt. Im Verlaufe von 1 Stunde setzt man 37,3 g (0,540 Mol) Natriumnitrat zu und rührt die Mischung für weitere 2 Stunden, bevor man sie im Autoklaven langsam auf 55 °C erhitzt. Anschliessend wird das überschüssige Fluorwasserstoff abdestilliert und der Rückstand in eine Mischung aus 200 ml Methylenchlorid und Eiswasser aufgenommen und mit konzentriertem Ammoniak neutralisiert. Durch dreimalige Extraktion mit Methylenchlorid, Waschen mit Wasser, Trocknen und Eindampfen der organischen Extrakte erhält man 81,0 g (48% d. Th.) 2-[4-(5-Chlor-3-fluorpyridin-2-yloxy)-phenoxy]-propionsäuremethylester, Smp. 63–64 °C.

d) 2-[4-(5-Chlor-3-fluorpyridin-2-yloxy)-phenoxy]-propionsäure.

Ein Gemisch aus 67,0 g (0,206 Mol) 2-[4-(5-Chlor-3-fluorpyridin-2-yloxy)-phenoxy]-propionsäuremethylester, 350 ml Dioxan und 250 ml 1N Natriumhydroxid-Lösung wird für 2 Stunden bei einer Temperatur von 40 °C gerührt. Das Reaktionsgemisch wird auf, eine Mischung aus Eis und 150 ml 2N Salzsäure gegossen und zweimal mit Äthylacetat extrahiert. Die Extrakte werden mit gesättigter Natriumchloridlösung gewaschen, über Magnesiumsulfat getrocknet, filtriert und eingedampft. Man erhält 63,6 g (99% d. Th.) 2-[4-(5-Chlor-3-fluorpyridin-2-yloxy)-phenoxy]-propionsäure, Smp. 95–97 °C.

e) 2-[4-(5-Chlor-3-fluorpyridin-2-yloxy)-phenoxy]-propionylchlorid.

63,6 g 2-[4-(5-Chlor-3-fluorpyridin-2-yloxy)-phenoxy]-propionsäure werden in 700 ml Toluol gelöst. Aus dieser Lösung werden 200 ml Toluol abdestilliert. Nach dem Abkühlen auf 90 °C setzt man tropfenweise 25 ml (0,34 Mol) Thionylchlorid zu und rührt die Lösung für weitere 14 Stunden bei dieser Temperatur. Durch Einengen verringert man das Volumen auf 200 ml. Die erhaltene toluolische Lösung von 2-[4-(5-Chlor-3-fluorpyridin-2-yloxy)-phenoxy]-propionylchlorid wird direkt in die folgende Reaktionsstufe eingesetzt.

f) Zu einer Lösung von 3,5 g (0,035 Mol) Triäthylamin und 3,2 g (0,033 Mol) n-Butylcyanamin in 40 ml Toluol lässt man unter Eiskühlung eine Lösung von 9,9 g (0,030 Mol) 2-[4-(5-Chlor-3-fluorpyridin-2-yloxy)-phenoxy]-propionylchlorid in 30 ml Toluol zutropfen. Nach einer Reaktionszeit von 5 Stunden trennt man den Niederschlag ab und reinigt das Filtrat durch Chromatographie an Kieselgel. Durch Eindampfen des Eluats erhält man 2-[4-(5-Chlor-3-fluorpyridin-2-yloxy)-phenoxy]-propionsäure-N-n-butyl-N-cyanamid in Form eines gelbstichigen Öles, $n_D^{25}$: 1,5482.

Beispiel H7

2R-[4-(5-Chlor-3-fluorpyridin-2-yloxy)-phenoxy]-propionsäure-N-äthyl-N-cyanamid.

Zu einer Lösung von 2,1 g (0,030 Mol) Äthylcyanamin und 9,9 g (0,030 Mol) 2R-[4-(5-Chlor-3-fluorpyridin-2-yloxy)-phenoxy]-propionylchlorid (Smp. 47–48 °C) in 30 ml Acetonitril wird unter Eiskühlung eine Lösung von 4,43 g (0,030 Mol) 1,8-Diazabicyclo[5.4.0]undec-7-en (97 proz.) in 10 Acetonitril getropft. Nach einer Reaktionszeit von 4 Std. wird das Reaktionsgemisch eingeengt. Das gewünschte 2R-[4-(5-Chlor-3-fluorpyridin-2-yloxy)-phenoxy]-propionsäure-N-äthyl-N-cyanamid erhält man durch Chromatographie an Kieselgel und Eindampfen des Eluates (Lösungsmittelgemisch: Äthylacetat/Hexan 1:1).

Beispiel H8

2-[4-(6-Fluor-chinoxalin-2-yloxy)-phenoxy]-propionsäure-N-cyan-N-äthyl-amid (Verb. Nr. 3.18).

10 g (0,039 Mol) 4-(6-Fluor-chinoxalin-2-yloxy)-phenol, 9,6 g (0,047 Mol) 2-Brompropionsäure-N-cyan-N-äthylamid und 8,1 g (0,058 Mol) Kaliumcarbonat werden in 100 ml Acetonitril für 14 Std. bei Raumtemperatur gerührt. Die ausgefallenen Salze werden abfiltriert, das Filtrat eingedampft. Der Rückstand wird an Kieselgel mit Äthylacetat/Hexan (1:3) chromatographiert. Man erhält 7,1 g (48% d. Th.) 2-[4-(6-Fluor-chinoxalin-2-yloxy)-phenoxy]-propionsäure-N-cyan-N-äthyl-amid, Smp. 108–111 °C.

In analoger Weise werden die in den folgenden Tabellen aufgelisteten Zwischenprodukte und Verbindungen der Formel I erhalten.

Tabelle 1

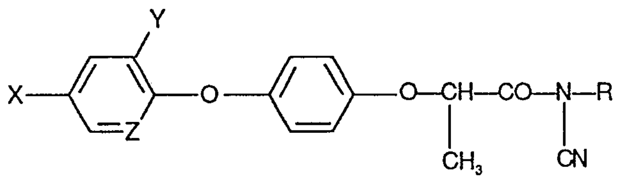

| Verb. Nr. | X | Y | Z | R | phys. Daten |
|---|---|---|---|---|---|
| 1.1 | $CF_3$ | H | N | $CH_3$ | $n_D^{23}$: 1.5210 |
| 1.2 | $CF_3$ | H | N | H | Smp.: 137-138 °C |
| 1.3 | $CF_3$ | H | CH | $CH_3$ | $n_D^{23}$: 1.5228 |
| 1.4 | $CF_3$ | H | CH | H | Smp.: 125-127 °C |
| 1.5 | Cl | Cl | N | $CH_3$ | Smp.: 89-90 °C |
| 1.6 | Cl | Cl | N | H | $n_D^{23}$: 1.5574 |
| 1.7 | Cl | Cl | N | $C_2H_5$ | |
| 1.8 | Cl | Cl | N | $C_4H_9$-n | |
| 1.9 | Cl | Cl | N | $-CH_2-CH=CH_2$ | |
| 1.10 | Cl | Cl | N | $-(CH_2)_2-OCH_3$ | |
| 1.11 | $CF_3$ | H | N | $C_2H_5$ | Smp.: 50-55 °C |
| 1.12 | $CF_3$ | H | N | $C_4H_9$-n | |
| 1.13 | $CF_3$ | H | N | $-CH_2-CH=CH_2$ | |
| 1.14 | $CF_3$ | H | N | $-(CH_2)_2-OCH_3$ | $n_D^{31}$: 1.5122 |
| 1.15 | $CF_3$ | H | CH | $C_2H_5$ | |
| 1.16 | $CF_3$ | H | CH | $C_4H_9$ | |
| 1.17 | $CF_3$ | H | CH | $-CH_2-CH=CH_2$ | Smp.: 79-80 °C |
| 1.18 | $CF_3$ | H | N | $-(CH_2)_2-OCH_3$ | |
| 1.19 | $CF_3$ | Cl | N | H | Smp.: 93-94 °C |
| 1.20 | $CF_3$ | Cl | N | $CH_3$ | Smp.: 94-95 °C |
| 1.21 | $CF_3$ | Cl | N | $C_2H_5$ | |
| 1.22 | $CF_3$ | Cl | N | $C_4H_9$-n | $n_D^{25}$: 1.5180 |
| 1.23 | $CF_3$ | Cl | N | $-CH_2-CH=CH_2$ | |
| 1.24 | $CF_3$ | Cl | N | $-(CH_2)_2-OCH_3$ | $n_D^{31}$: 1.5162 |
| 1.25 | Cl | F | N | H | |
| 1.26 | Cl | F | N | $CH_3$ | |
| 1.27 | Cl | F | N | $C_2H_5$ | $n_D^{25}$: 1.5545 |
| 1.28 | Cl | F | N | $C_4H_9$-n | $n_D^{25}$: 1.5482 |
| 1.29 | Cl | F | N | $-CH_2-CH=CH_2$ | |
| 1.30 | Cl | F | N | $-(CH_2)_2-OCH_3$ | |

Tabelle 2

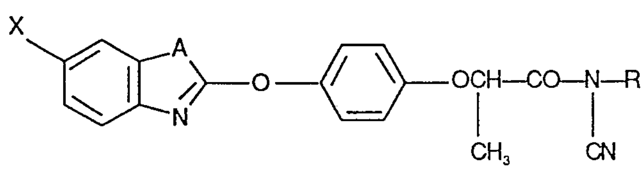

| Verb. Nr. | A | X | R | phys. Daten |
|---|---|---|---|---|
| 2.1 | O | Cl | H | |
| 2.2 | O | Cl | $CH_3$ | |
| 2.3 | O | Cl | $C_2H_5$ | |
| 2.4 | O | Cl | $C_4H_9$-n | Smp. 91-92 °C |
| 2.5 | O | Cl | $-CH_2-CH=CH_2$ | |
| 2.6 | O | Cl | $-(CH_2)_2-OCH_3$ | |
| 2.7 | O | Cl | $-CH_2-C{\equiv}CH$ | |
| 2.8 | S | Cl | H | |
| 2.9 | S | Cl | $CH_3$ | |

Tabelle 2 (Fortsetzung)

| Verb. Nr. | A | X | R | phys. Daten |
|---|---|---|---|---|
| 2.10 | S | Cl | $C_2H_5$ | |
| 2.11 | S | Cl | $C_4H_9$-n | |
| 2.12 | S | Cl | $-CH_2-CH=CH_2$ | |
| 2.13 | S | Cl | $-(CH_2)_2-OCH_3$ | |
| 2.14 | O | F | H | |
| 2.15 | O | F | $CH_3$ | |
| 2.16 | S | F | H | |
| 2.17 | S | F | $CH_3$ | |
| 2.18 | O | $CF_3$ | H | |
| 2.19 | O | $CF_3$ | $CH_3$ | |

Tabelle 3

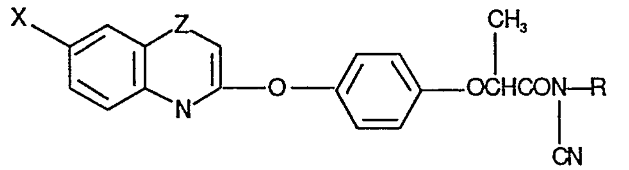

| Verb. Nr. | X | Z | R | phys. Daten |
|---|---|---|---|---|
| 3.1 | Cl | N | H | Smp.: 152–154 °C |
| 3.2 | Cl | N | $CH_3$ | Smp.: 106–108 °C |
| 3.3 | Cl | N | $C_2H_5$ | Smp.: 106–111 °C |
| 3.4 | Cl | N | $C_4H_9$-n | Smp.: 62–65 °C |
| 3.5 | Cl | N | $-CH_2-CH=CH_2$ | |
| 3.6 | Cl | N | $-(CH_2)_2-OCH_3$ | $n_D^{40}$: 1.5794 |
| 3.7 | Cl | CH | H | |
| 3.8 | Cl | CH | $CH_3$ | |
| 3.9 | Cl | CH | $C_2H_5$ | |
| 3.10 | Cl | CH | $C_4H_9$-n | |
| 3.11 | Cl | CH | $-CH_2-CH=CH_2$ | |
| 3.12 | Cl | CH | $-(CH_2)_2-OCH_3$ | |
| 3.13 | F | N | H | Smp.: 167–169 °C |
| 3.14 | F | N | $CH_3$ | Smp.: 141–143 °C |
| 3.15 | F | CH | H | |
| 3.16 | F | CH | $CH_3$ | |
| 3.17 | F | N | $C_4H_9$-n | Smp.: 94–95 °C |
| 3.18 | F | N | $C_2H_5$ | Smp.: 108–111 °C |

Tabelle 4

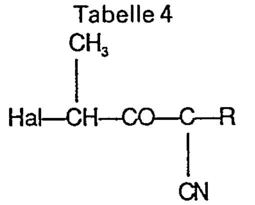

| Verb. Nr. | Hal | R | phys. Daten |
|---|---|---|---|
| 4.1 | Br | H | |
| 4.2 | Br | $CH_3$ | Sdp. 45–46 °C/0.008 mbar |
| 4.3 | Cl | H | |
| 4.4 | Cl | $CH_3$ | |
| 4.5 | Cl | $C_4H_9$-n | |
| 4.6 | Br | $C_4H_9$-n | Sdp. 53–54 °C/0.0052 mbar |
| 4.7 | Br | $C_2H_5$ | Sdp. 40–45 °C/0,0075 mbar |
| 4.8 | Br | $-(CH_2)_2-OCH_3$ | Sdp. 85 °C/0.02 mbar |

Tabelle 5

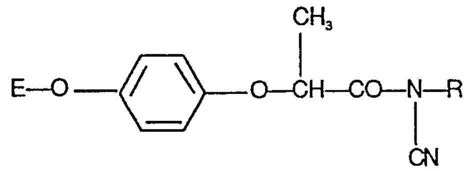

| Verb. Nr. | E | R | phys. Daten |
|---|---|---|---|
| 5.1 | Benzyl | H | |
| 5.2 | Benzyl | $CH_3$ | Smp. 56-58 °C |
| 5.3 | Benzyl | $C_2H_5$ | Smp. 57-63 °C |
| 5.4 | Benzyl | $C_4H_9$-n | Smp. 108-109 °C |
| 5.5 | H | H | |
| 5.6 | H | $CH_3$ | Öl |
| 5.7 | H | $C_2H_5$ | |
| 5.8 | H | $C_4H_9$-n | $n_D^{25}$: 1.5132 |

Tabelle 6

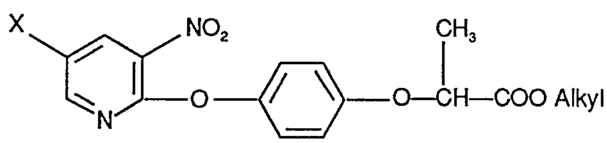

| Verb. Nr. | X | Alkyl | phys. Daten |
|---|---|---|---|
| 6.1 | Cl | $CH_3$ | Smp. 91-92 °C |
| 6.2 | Br | $CH_3$ | |
| 6.3 | F | $CH_3$ | |
| 6.4 | Cl | $C_2H_5$ | |
| 6.5 | Cl | $C_4H_9$-n | |
| 6.6 | $CF_3$ | $CH_3$ | |
| 6.7 | $CF_3$ | $C_4H_9$-n | |

Tabelle 7

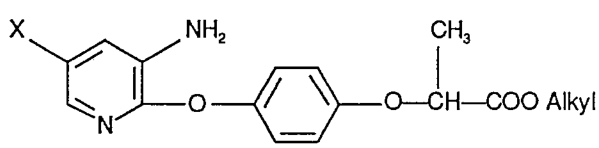

| Verb. Nr. | X | Alkyl | phys. Daten |
|---|---|---|---|
| 7.1 | Cl | $CH_3$ | Smp. 92-94 °C |
| 7.2 | Br | $CH_3$ | |
| 7.3 | F | $CH_3$ | |
| 7.4 | Cl | $C_2H_5$ | |
| 7.5 | Cl | $C_4H_9$-n | Öl |
| 7.6 | $CF_3$ | $CH_3$ | |
| 7.7 | $CF_3$ | $C_4H_9$-n | |

Tabelle 8

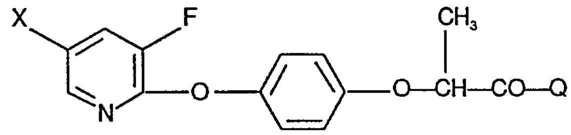

| Verb. Nr. | X | Q | phys. Daten |
|---|---|---|---|
| 8.1 | Cl | OH | Smp. 95-97 °C |
| 8.2 | Cl | ONa | |
| 8.3 | Cl | OK | |
| 8.4 | Br | OH | |
| 8.5 | F | OH | |
| 8.6 | $CF_3$ | OH | |
| 8.7 | Cl | Cl | Öl |
| 8.8 | Cl | Br | |
| 8.9 | Cl | F | |
| 8.10 | Br | Cl | |
| 8.11 | F | Cl | |
| 8.12 | $CF_3$ | Cl | |

Tabelle 9

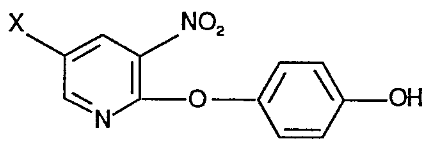

| Verb. Nr. | X | phys. Daten |
|---|---|---|
| 9.1 | Cl | Smp. 125-126 °C |
| 9.2 | Br | |
| 9.3 | F | |
| 9.4 | $CF_3$ | |

Tabelle 10

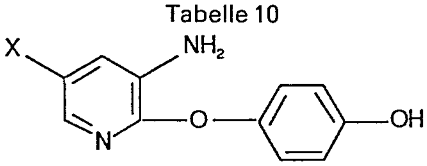

| Verb. Nr. | X | phys. Daten |
|---|---|---|
| 10.1 | Cl | Smp. 174 °C |
| 10.2 | Br | |
| 10.3 | F | |
| 10.4 | $CF_3$ | |

Tabelle 11

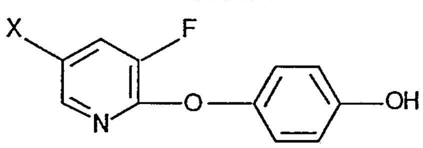

| Verb. Nr. | X | phys. Daten |
|---|---|---|
| 11.1 | Cl | Smp. 97-98 °C |
| 11.2 | Br | |
| 11.3 | F | |
| 11.4 | $CF_3$ | |

## Formulierungsbeispiele

### Beispiel F1

Formulierung flüssiger Wirkstoffe der Formel I (% = Gewichtsprozent)

| a) Emulsionskonzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylen-glykoläther (36 Mol AeO) | 5% | - | - |
| Tributylphenolpolyäthylen-glykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff | 80% | 10% | 5% | 95% |
| Äthylenglykol-monomethyl-äther | 20% | - | - | - |
| Polyäthylenglykol MG 400 | - | 70% | - | - |
| N-Methyl-2-pyrrolidon | - | 20% | - | - |
| Epoxidiertes Kokosnussöl | - | - | 1% | 5% |
| Benzin (Siedegrenzen 160-190 °C) | - | - | 94% | - |

Die Lösungen sind zur Anwendung in Form kleinster Tropfen geeignet.

| c) Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff | 2% | 5% |
| Hochdisperse Kieselsäure | 1% | 5% |

| | | |
|---|---|---|
| Talkum | 97% | - |
| Kaolin | - | 90% |

Durch inniges Vermischen der Trägerstoffe mit dem Wirkstoff erhält man gebrauchsfertige Stäubemittel.

Beispiel F2

Formulierung fester Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | - | - |
| Na-Diisobutyl-naphthalinsulfonat | - | 6% | 6% |
| Octylphenolpolyäthylenglykoläther (7–8 Mol AeO) | - | 2% | 2% |
| Hochdisperse Kieselsäure | 5% | 27% | 27% |
| Kaolin | 67% | - | - |
| Natriumchlorid | - | - | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10% | 1% |
| Octylphenolpoly äthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) | c) |
|---|---|---|---|
| Wirkstoff | 5% | 8% | 0,1% |
| Talkum | 95% | - | 99,9% |
| Kaolin | - | 92% | - |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyäthylenglykol (MG 200) | 3% |
| Kaolin | 96% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40% | 5% |
| Äthylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Zu Vergleichszwecken sind den Resultaten der biologischen Beispiele B1 und B2 jeweils Versuchsergebnisse mit der Verbindung A

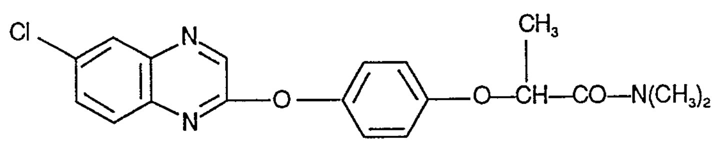

(A)

die aus der DE-OS 3 004 770, Seite 12, Nr. 58, bekannt ist, angefügt.

Beispiel B1

Preemergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat, bzw. aus einem 25%igen Spritzpulver bei Wirkstoffen, die wegen ungenügender Löslichkeit nicht als Emulsionskonzentrat formuliert werden können, behandelt. Es wurden zwei verschiedene Konzentrationsreihen angewendet, entsprechend 1 und 0,5 kg Wirksubstanz pro Hektar. Die Saatschalen werden im Gewächshaus bei 22–25°C und 50–70% rel. Luftfeuchtigkeit gehalten und der Versuch nach 3 Wochen nach der folgenden Bewertungsskala ausgewertet:

1 = Pflanzen nicht gekeimt oder total abgestorben

2–3 = sehr starke Wirkung
4–6 = mittlere Wirkung
7–8 = geringe Wirkung
9 = keine Wirkung

Die geprüften Verbindungen der Formel I zeigten in diesem Versuch ausgezeichnete Wirkung gegen einkeimblättrige grasartige Unkräuter, während Kulturpflanzen wie Weizen, Gerste, Zuckerrüben, Soja und Baumwolle bei der angewandten Aufwandmenge nicht oder nur unbedeutend geschädigt wurden. Die Vergleichsverbindung A ist nahezu unwirksam.

Resultate

| Testpflanze | Verbindung Nr. | | | | | | | |
|---|---|---|---|---|---|---|---|---|
| | 1.1 kg/ha | | 1.2 kg/ha | | 1.3 kg/ha | | 1.4 kg/ha | |
| | 1 | 0,5 | 1 | 0,5 | 1 | 0,5 | 1 | 0,5 |
| Lolium | 1 | 1 | 1 | 2 | 1 | 2 | 1 | 2 |
| Alopecurus | 1 | 1 | 1 | 1 | 1 | 2 | 2 | 2 |
| Digitaria | 1 | 1 | 1 | 1 | 1 | 1 | 1 | 1 |
| Echinochloa | 1 | 1 | 1 | 2 | 1 | 1 | 1 | 1 |
| Sorghum | 1 | 1 | 1 | 1 | 1 | 1 | 1 | 2 |
| Rottboellia | 1 | 1 | 1 | 1 | 1 | 1 | 1 | 1 |
| Soja | 9 | 9 | 7 | 7 | 9 | 9 | 9 | 9 |
| Baumwolle | 9 | 9 | 9 | 9 | 9 | 9 | 9 | 9 |
| Zuckerrüben | 9 | 9 | 9 | 9 | 9 | 9 | 9 | 9 |

Resultate (Fortsetzung)

| Testpflanze | Verbindung Nr. | | | |
|---|---|---|---|---|
| | 1.5 kg/ha | | 1.6 kg/ha | |
| | 1 | 0,5 | 1 | 0,5 |
| Lolium | 1 | 1 | 1 | 3 |
| Alopecurus | 1 | 2 | 2 | 4 |
| Digitaria | 1 | 1 | 1 | 1 |
| Echinochloa | 1 | 1 | 1 | 3 |
| Sorghum | 1 | 1 | 1 | 2 |
| Rottboellia | 1 | 1 | 2 | 2 |
| Gerste | 7 | 9 | 7 | 8 |
| Weizen | 6 | 8 | 8 | 9 |

| Testpflanze | Verbindung Nr. | | | | | | | | | |
|---|---|---|---|---|---|---|---|---|---|---|
| | 3.3 kg/ha | | 3.4 kg/ha | | 3.17 kg/ha | | 3.18 kg/ha | | A kg/ha | |
| | 1 | 0,5 | 1 | 0,5 | 1 | 0,5 | 1 | 0,5 | 1 | 0,5 |
| Lolium | 7 | 9 | 5 | 8 | 3 | 3 | 2 | 4 | 9 | 9 |
| Alopecurus | 4 | 4 | 3 | 4 | 1 | 2 | 1 | 2 | 8 | 9 |
| Digitaria | 2 | 4 | 1 | 1 | 1 | 1 | 1 | 1 | 4 | 6 |
| Echinochloa | 2 | 2 | 2 | 6 | 1 | 1 | 1 | 1 | 9 | 9 |
| Sorghum | 1 | 3 | 2 | 7 | 1 | 3 | 1 | 2 | 9 | 9 |
| Rottboellia | 2 | 6 | 3 | 7 | 4 | 4 | 1 | 2 | 8 | 9 |
| Gerste | 9 | 9 | 8 | 9 | 8 | 9 | 8 | 9 | 9 | 9 |
| Weizen | 8 | 9 | 7 | 9 | 4 | 4 | 3 | 5 | 9 | 9 |

Beispiel B2

Postemergente Herbizid-Wirkung (Kontaktherbizid)
Eine Anzahl Unkräuter und Kulturpflanzen, sowohl monokotyle wie dikotyle, wurden nach dem Auflaufen (in 4-bis-6 Blattstadium) mit einer wässrigen Wirkstoffdispersion in Dosierungen von 0,25, 0,5 und 1kg Wirksubstanz pro Hektar auf die Pflanzen gespritzt und diese bei 24–26 °C und 45–60% relativer Luftfeuchtigkeit gehalten. Mindestens 15 Tage nach der Behandlung wird der Versuch nach der gleichen Bewertungsskala wie beim preemergenten Test ausgewertet.

Auch in diesem Versuch zeigten die Verbindungen der Formel I ausgezeichnete Wirkung gegen einkeimblättrige grasartige Unkräuter wobei die Kulturpflanze wie Weizen, Gerste, Zuckerrüben, Baumwolle und Soja nicht oder erst mit höheren Aufwendungen an Wirkstoff geschädigt wurden. Die Vergleichsverbindung A ist nahezu unwirksam.

Resultate

| Testpflanze | Verbindung Nr. | | | | | | | |
|---|---|---|---|---|---|---|---|---|
| | 1.1 kg/ha | | 1.2 kg/ha | | 1.3 kg/ha | | 1.4 kg/ha | |
| | 1 | 0,5 | 1 | 0,5 | 1 | 0,5 | 1 | 0,5 |
| Lolium | 1 | 2 | 2 | 3 | 2 | 2 | 3 | 3 |
| Alopecurus | 1 | 2 | 2 | 3 | 1 | 2 | 2 | 6 |
| Digitaria | 1 | 1 | 1 | 1 | 1 | 1 | 1 | 1 |
| Echinochloa | 1 | 1 | 1 | 1 | 1 | 1 | 1 | 1 |
| Sorghum | 1 | 1 | 1 | 1 | 1 | 1 | 1 | 2 |
| Rottboellia | 1 | 1 | 1 | 2 | 1 | 1 | 1 | 2 |
| Soja | 9 | 9 | 9 | 9 | 6 | 9 | 9 | 9 |
| Baumwolle | 7 | 9 | 9 | 9 | 8 | 8 | 9 | 9 |
| Zuckerrüben | 8 | 9 | 8 | 9 | 8 | 8 | 8 | 9 |

| Testpflanze | Verbindung Nr. | | | |
|---|---|---|---|---|
| | 1.5 kg/ha | | 1.6 kg/ha | |
| | 1 | 0,5 | 1 | 0,5 |
| Avena | 1 | 2 | 2 | 5 |
| Lolium | 2 | 2 | 4 | 5 |
| Alopecurus | 1 | 2 | 4 | 8 |
| Digitaria | 1 | 1 | 1 | 2 |
| Echinochloa | 1 | 1 | 1 | 1 |
| Sorghum | 1 | 1 | 1 | 3 |
| Rottboellia | 1 | 1 | 1 | 3 |
| Gerste | 3 | 7 | 9 | 9 |
| Weizen | 8 | 9 | 9 | 9 |

| Testpflanze | Verbindung Nr. | | | | | | | | | |
|---|---|---|---|---|---|---|---|---|---|---|
| | 3.3 kg/ha | | 3.4 kg/ha | | 3.17 kg/ha | | 3.18 kg/ha | | A kg/ha | |
| | 0,5 | 0,25 | 0,5 | 0,25 | 0,5 | 0,25 | 0,5 | 0,25 | 0,5 | 0,25 |
| Lolium | 1 | 2 | 3 | 3 | 1 | 1 | 1 | 2 | 7 | 9 |
| Alopecurus | 2 | 2 | 1 | 1 | 1 | 1 | 1 | 2 | 9 | 9 |
| Digitaria | 1 | 1 | 1 | 1 | 1 | 1 | 1 | 1 | 5 | 8 |
| Echinochloa | 1 | 1 | 1 | 1 | 1 | 1 | 1 | 1 | 8 | 9 |
| Sorghum | 1 | 1 | 1 | 1 | 1 | 1 | 1 | 1 | 8 | 9 |
| Rottboellia | 1 | 1 | 1 | 1 | 1 | 1 | 1 | 1 | 6 | 9 |
| Soja | 9 | 9 | 7 | 8 | 8 | 8 | 8 | 9 | 7 | 9 |
| Baumwolle | 8 | 9 | 8 | 9 | 7 | 9 | 8 | 8 | 7 | 9 |
| Zuckerrüben | 8 | 9 | 8 | 9 | 7 | 8 | 8 | 8 | 8 | 9 |

**Patentansprüche für die Vertragsstaaten: BE, CH, DE, FR, GB, IT, LI, NL, SE**

1. 2-Phenoxypropionsäure-cyanamide der Formel I

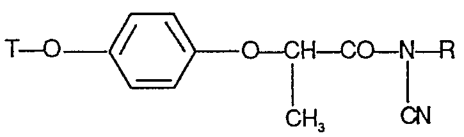

(I)

worin

R Wasserstoff, $C_1$-$C_4$-Alkyl, $C_3$-$C_4$-Alkenyl, $C_3$-$C_4$-Alkinyl oder $C_2$-$C_4$-Alkoxyalkyl bedeutet und

T für einen Rest

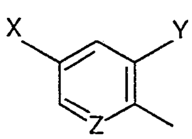

,

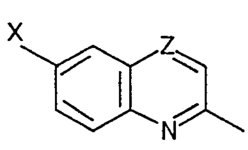

oder

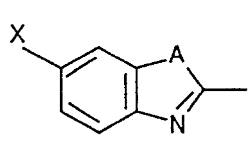

steht, in welchen

A Sauerstoff oder Schwefel,

X Fluor, Chlor, Brom, Jod oder Trifluormethyl,

Y Wasserstoff, Fluor, Chlor, Brom oder Trifluormethyl und

Z Stickstoff oder die Methinbrücke bedeuten.

2. Verbindungen gemäss Anspruch 1, dadurch gekennzeichnet, dass R Wasserstoff oder $C_1$-$C_4$-Alkyl bedeutet.

3. Verbindungen gemäss Anspruch 2, dadurch gekennzeichnet, dass R $C_1$-$C_4$-Alkyl bedeutet.

4. Verbindungen gemäss Anspruch 1, dadurch gekennzeichnet, dass T für den Rest

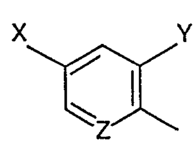

steht, worin X, Y und Z die in Anspruch 1 gegebene Bedeutung haben.

5. Verbindungen gemäss Anspruch 1, dadurch gekennzeichnet, dass T für den Rest

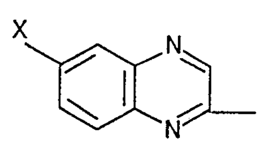

steht, worin X die unter Anspruch 1 gegebene Bedeutung hat.

6. Verbindung gemäss Anspruch 5, dadurch gekennzeichnet, dass X Fluor oder Chlor bedeutet.

7. Verbindungen gemäss Anspruch 1, dadurch gekennzeichnet, dass T für den Rest

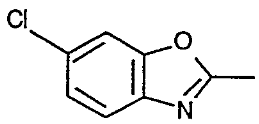

,

steht.

8. Verbindungen gemäss Anspruch 4, dadurch gekennzeichnet, dass X für Chlor, Y für Fluor und Z für Stickstoff stehen.

9. Verbindungen gemäss Anspruch 1, dadurch gekennzeichnet, dass R für $C_1$–$C_4$-Alkyl und T für 6-Fluor-chinoxalin-2-yl stehen.

10. Verbindungen gemäss Anspruch 1, dadurch gekennzeichnet, dass R für $C_1$–$C_4$-Alkyl und T für 6-Chlor-chinoxalin-2-yl stehen.

11. Verbindungen gemäss Anspruch 1, dadurch gekennzeichnet, dass R für $C_1$–$C_4$-Alkyl und T für 6-Chlor-benzoxazol-2-yl stehen.

12. Verbindungen gemäss Anspruch 1, dadurch gekennzeichnet, dass R für $C_1$–$C_4$-Alkyl und T für 5-Chlor-3-fluorpyridin-2-yl stehen.

13. 2-[4-(5-Chlor-3-fluorpyridin-2-yloxy)-phenoxy]-propionsäure-N-cyan-N-methyl-amid gemäss Anspruch 1.

14. 2-[4-(5-Trifluormethylpyridin-2-yloxy)-phenoxy]-propionsäure-N-cyan-N-methyl-amid gemäss Anspruch 1.

15. 2-[4-(5-Chlor-3-fluorpyridin-2-yloxy)-phenoxy]-propionsäure-N-cyan-N-äthyl-amid gemäss Anspruch 1.

16. 2-[4-(6-Chlor-chinoxalin-2-yloxy)-phenoxy]-propionsäure-N-cyan-N-äthyl-amid gemäss Anspruch 1.

17. 2-[4-(6-Chlor-chinoxalin-2-yloxy)-phenoxy]-propionsäure-N-cyan-N-methyl-amid gemäss Anspruch 1.

18. 2-[4-(6-Fluor-chinoxalin-2-yloxy)-phenoxy]-phenoxy]-propionsäure-N-cyan-N-äthyl-amid gemäss Anspruch 1.

19. 2-[4-(6-Fluor-chinoxalin-2-yloxy)-phenoxy]-propionsäure-N-cyan-N-methyl-amid gemäss Anspruch 1.

20. 2-[4-(6-Chlor-benzoxazol-2-yloxy)-phenoxy]-propionsäure-N-cyan-N-n-butyl-amid gemäss Anspruch 1.

21. Verfahren zur Herstellung der Verbindungen der Formel I gemäss Anspruch 1, dadurch gekennzeichnet, dass man ein 2-Phenoxypropionsäurehalogenid der Formel II

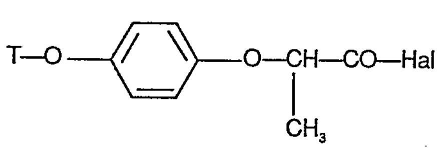

(II),

worin T die unter Formel I gegebene Bedeutung hat und Hal für Chlor oder Brom steht, in Gegenwart eines säurebindenden Mittels mit einem Cyanamin der Formel III

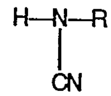

(III),

worin R die im Anspruch 1 unter Formel I gegebene Bedeutung hat, umsetzt.

22. Verfahren zur Herstellung der Verbindungen der Formel Ia

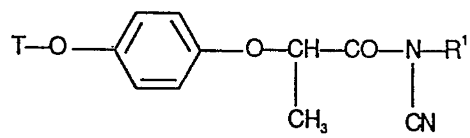

(Ia),

worin T die in Anspruch 1 unter Formel I gegebene Bedeutung hat und $R^1$ für $C_1$–$C_4$-Alkyl, $C_3$–$C_4$-Alkenyl, $C_3$–$C_4$-Alkinyl oder $C_2$–$C_4$-Alkoxyalkyl steht, dadurch gekennzeichnet, dass man eine entsprechende Cyanamino-Verbindung der Unterformel Ib

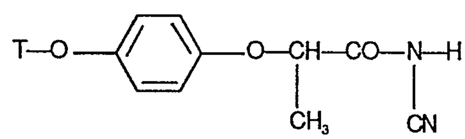

(Ib),

worin T die in Anspruch 1 unter Formel I gegebene Bedeutung hat, in Gegenwart eines säurebindenden Mittels mit einer Halogenverbindung der Formel IV

$$Hal\text{-}R^1 \qquad (IV)$$

worin $R^1$ die unter Formel Ia gegebene Bedeutung hat und Hal für Chlor, Brom oder Jod steht, umsetzt.

23. Verfahren zur Herstellung der Verbindungen der Formel I, dadurch gekennzeichnet, dass man ein Phenoxypropionsäurederivat der Formel V

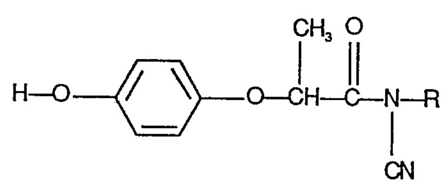

(V)

worin R die unter Formel I gegebene Bedeutung hat, in Gegenwart eines säurebindenden Mittels mit einem Halogenid der Formel VI

$$T\text{-}Hal \qquad (VI)$$

worin T die unter Formel I gegebene Bedeutung hat und Hal für Fluor, Chlor, Brom oder Jod steht, umsetzt.

24. Verfahren zur Herstellung der Verbindungen der Formel I, dadurch gekennzeichnet, dass man ein Propionsäurederivat der Formel VII

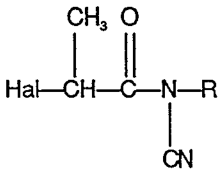

(VII)

worin R die unter Formel I gegebene Bedeutung hat und Hal für Chlor, Brom, Tosyl oder Mesyl steht, in Gegenwart eines säurebindenden Mittels mit einem Hydrochinonderivat der Formel VIII

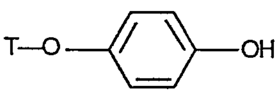

(VIII)

worin T die unter Formel I gegebene Bedeutung hat, umsetzt.

25. Ein herbizides Mittel, dadurch gekennzeichnet, dass es neben Träger- und/oder anderen Zuschlagstoffen als Wirkstoff mindestens ein 2-Phenoxypropionsäureamid der Formel I, gemäss Anspruch 1, enthält.

26. Die Verwendung der Wirkstoffe der Formel I, gemäss Anspruch 1, oder sie enthaltender Mittel zur Bekämpfung von Unkräutern.

27. Die Verwendung gemäss Anspruch 26, zur selektiven Unkrautbekämpfung.

28. Die Verwendung gemäss Anspruch 27, zur selektiven Bekämpfung von monokotylen Unkräutern.

29. Die Verwendung gemäss Anspruch 28, zur selektiven pre- oder postemergenten Bekämpfung von monokotylen Unkräutern in Nutzpflanzenkulturen.

30. Die Verwendung gemäss Anspruch 29 in dikotylen Nutzpflanzenkulturen wie Soja, Raps, Zuckerrüben und Baumwolle.

31. Verbindungen der Formel VII

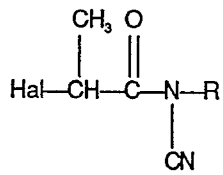

(VII)

worin R die unter Formel I im Anspruch 1 gegebene Bedeutung hat und Hal für Chlor, Brom, Tosyl oder Mesyl steht.

32. Verbindungen der Formel V

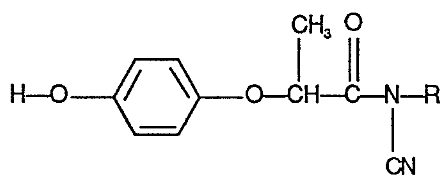

(V)

worin R die unter Formel I im Anspruch 1 gegebene Bedeutung hat.

33. Verbindungen der Formel XII

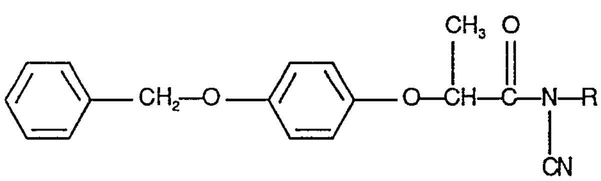

(XII)

worin R die unter Formel I im Anspruch 1 gegebene Bedeutung hat.

34. Verfahren zur Herstellung der Verbindungen der Formel Ic

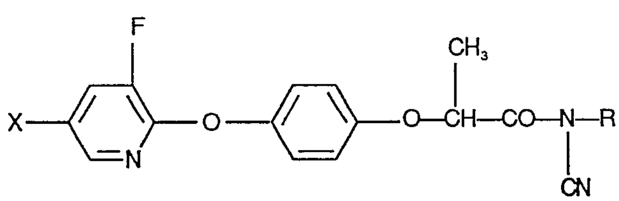

(Ic)

worin R und X die unter Formel I gegebene Bedeutung haben, dadurch gekennzeichnet, dass man entweder

a) ein 3-Nitropyridin der Formel XIII

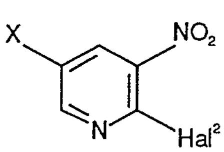

(XIII)

worin X die unter Formel I gegebene Bedeutung hat und $Hal^2$ für Fluor, Chlor oder Brom steht, in Gegenwart einer Base mit Hydrochinon zu einer Verbindung der Formel XIV

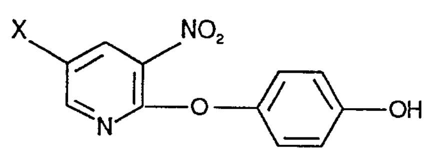

(XIV)

umsetzt, dieses Zwischenprodukt in Gegenwart eines Metallkatalysators zu einer Aminoverbindung der Formel XV

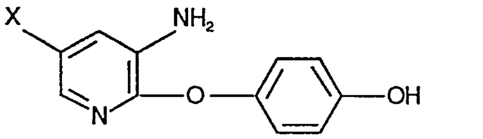

(XV)

reduziert, diese diazotiert und mit einem Fluorierungsmittel in eine Verbindung der Formel XVI

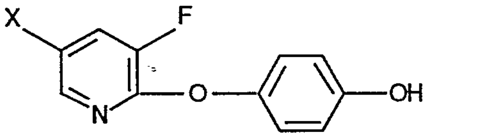

(XVI)

überführt und dieses fluorierte Zwischenprodukt in Gegenwart einer Base mit einem Propinsäure-Derivat der Formel VII umsetzt, oder dass man

b) das Nitropyridin der Formel (XIII) in Gegenwart einer Base mit einem Propionsäureester der Formel XVII

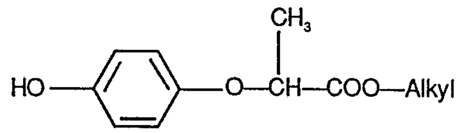

(XVII)

worin Alkyl für $C_1$-$C_4$-Alkyl steht, umsetzt, das erhaltene Zwischenprodukt der Formel XVIII

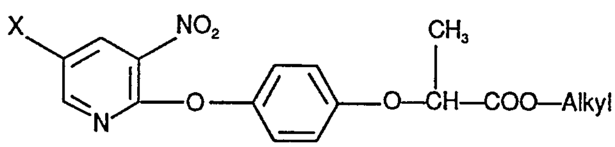

(XVIII)

in Gegenwart eines Metallkatalysators zur Aminoverbindung der Formel XIX

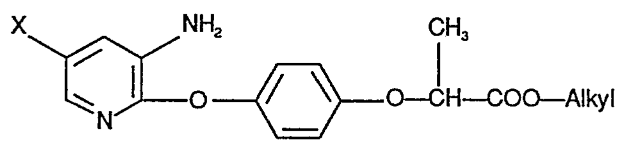

(XIX)

reduziert, dieses Zwischenprodukt diazotiert und mit einem Fluorierungsmittel in eine Verbindung der Formel XX

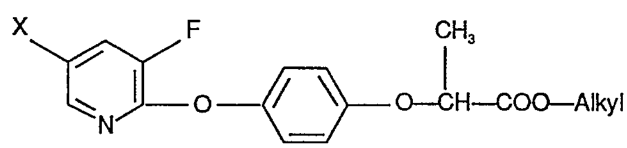

(XX)

überführt, diesen Ester verseift und mit einem Halogenierungsmittel in ein 2-Phenoxypropionsäurehalogenid der Unterformel IIa

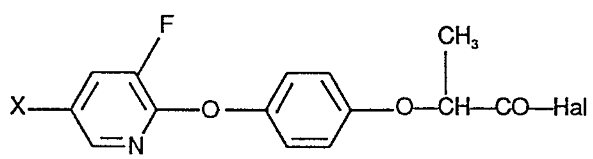

(IIa)

worin Hal und X die unter Formel II gegebene Bedeutung haben, überführt und in Gegenwart einer Base mit einem Cyanamin der Formel III umsetzt.

**Patentansprüche für den Vertragsstaat: AT**

1. Ein herbizides Mittel, dadurch gekennzeichnet, dass es neben Träger- und/oder anderen Zuschlagstoffen mindestens ein 2-Phenoxypropionsäure-cyanamide der Formel I

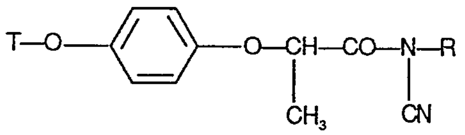

(I)

worin

R Wasserstoff, $C_1$-$C_4$-Alkyl, $C_3$-$C_4$-Alkenyl, $C_3$-$C_4$-Alkinyl oder $C_2$-$C_4$-Alkoxyalkyl bedeutet und

T für einen Rest

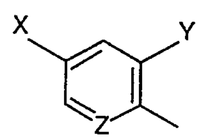

,

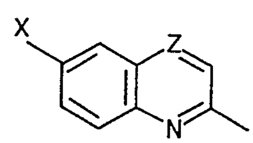

oder

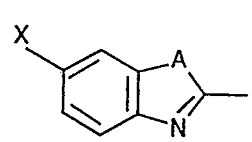

steht, in welchen
A Sauerstoff oder Schwefel,
X Fluor, Chlor, Brom, Jod oder Trifluormethyl,
Y Wasserstoff, Fluor, Chlor, Brom oder Trifluormethyl und
Z Stickstoff oder die Methinbrücke bedeuten.

2. Mittel gemäss Anspruch 1, dadurch gekennzeichnet, dass R Wasserstoff oder $C_1$-$C_4$-Alkyl bedeutet.

3. Mittel gemäss Anspruch 2, dadurch gekennzeichnet, dass R $C_1$-$C_4$-Alkyl bedeutet.

4. Mittel gemäss Anspruch 1, dadurch gekennzeichnet, dass T für den Rest

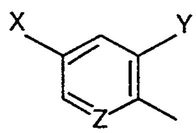

steht, worin X, Y und Z die in Anspruch 1 gegebene Bedeutung haben.

5. Mittel gemäss Anspruch 1, dadurch gekennzeichnet, dass T für den Rest

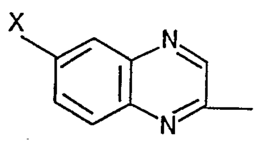

steht, worin X die in Anspruch 1 gegebene Bedeutung hat.

6. Mittel gemäss Anspruch 5, dadurch gekennzeichnet, dass X Fluor oder Chlor bedeutet.

7. Mittel gemäss Anspruch 1, dadurch gekennzeichnet, dass T für den Rest

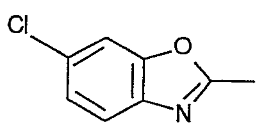

steht.

8. Mittel gemäss Anspruch 4, dadurch gekennzeichnet, dass X für Chlor, Y für Fluor und Z für Stickstoff stehen.

9. Mittel gemäss Anspruch 1, dadurch gekennzeichnet, dass R für $C_1$-$C_4$-Alkyl und T für 6-Fluorchinoxalin-2-yl stehen.

10. Mittel gemäss Anspruch 1, dadurch gekennzeichnet, dass R für $C_1$-$C_4$-Alkyl und T für 6-Chlor-chinoxalin-2-yl stehen.

11. Mittel gemäss Anspruch 1, dadurch gekennzeichnet, dass R für $C_1$-$C_4$-Alkyl und T für 6-Chlor-benzoxazol-2-yl stehen.

12. Mittel gemäss Anspruch 1, dadurch gekennzeichnet, dass R für $C_1$-$C_4$ Alkyl und T für 5-Chlor-3-fluorpyridin-2-yl stehen.

13. Mittel gemäss Anspruch 1, dadurch gekennzeichnet, dass es einen Wirkstoff aus der Gruppe 2-[4-(5-Chlor-3-fluorpyridin-2-yloxy)-phenoxy]-propionsäure-N-cyan-N-methyl-amid, 2-[4-(5-Trifluormethylpyridin-2-yloxy)-phenoxy]-propionsäure-N-cyan-N-methyl- amid, 2-[4-(5-Chlor-3-fluor-pyridin-2-yloxy)-phenoxy]-propionsäure-N-cyan-N-äthyl-amid, 2-[4-(6-Chlor-chinoxalin-2-yloxy)-phenoxy]-propionsäure-N-cyan-N-äthyl-amid, 2-[4-(6-Chlor-chinoxalin-2-yloxy)-phenoxy]-propionsäure-N-cyan-N-methyl-amid, 2-[4-(6-Fluor-chinoxalin-2-yloxy)-phenoxy]-propionsäure-N-cyan-N-äthyl-amid, 2-[4-(6-Fluor-chinoxalin-2-yloxy)-phenoxy]-propionsäure-N-cyan-N-methyl-amid und 2-[4-(6-Chlor-benzoxazol-2-yloxy)-phenoxy]-propionsäure-N-cyan-N-n-butyl-amid enthält.

14. Verfahren zur Herstellung der Verbindungen der Formel I gemäss Anspruch 1, dadurch gekennzeichnet, dass man ein 2-Phenoxypropionsäurehalogenid der Formel II

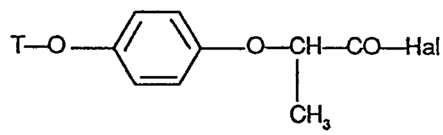 (II),

worin T die unter Formel I gegebene Bedeutung hat und Hal für Chlor oder Brom steht, in Gegenwart eines säurebindenden Mittels mit einem Cyanamin der Formel III

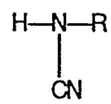 (III),

worin R die im Anspruch 1 unter Formel I gegebene Bedeutung hat, umsetzt.

15. Verfahren zur Herstellung der Verbindung der Formel Ia

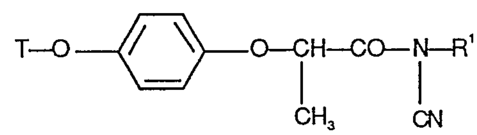 (Ia),

worin T die in Anspruch 1 unter Formel I gegebene Bedeutung hat und $R^1$ für $C_1$-$C_4$-Alkyl, $C_3$-$C_4$-Alkenyl, $C_3$-$C_4$-Alkinyl oder $C_2$-$C_4$-Alkoxyalkyl steht, dadurch gekennzeichnet, dass man eine entsprechende Cyanamino-Verbindung der Unterformel Ib

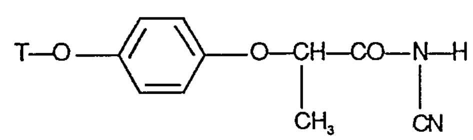 (Ib),

worin T die in Anspruch 1 unter Formel I gegebene Bedeutung hat, in Gegenwart eines säurebindenden Mittels mit einer Halogenverbindung der Formel IV

Hal-$R^1$ (IV)

worin $R^1$ die unter Formel Ia gegebene Bedeutung hat und Hal für Chlor, Brom oder Jod steht, umsetzt.

16. Verfahren zur Herstellung der Verbindungen der Formel I, dadurch gekennzeichnet, dass man ein Phenoxypropionsäurederivat der Formel V

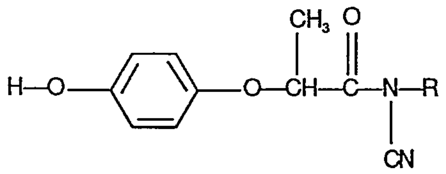 (V)

worin R die unter Formel I gegebene Bedeutung hat, in Gegenwart eines säurebindenden Mittels mit einem Halogenid der Formel VI

T-Hal (VI)

worin T die unter Formel I gegebene Bedeutung hat und Hal für Fluor, Chlor, Brom oder Jod steht, umsetzt.

17. Verfahren zur Herstellung der Verbindungen der Formel I, dadurch gekennzeichnet, dass man ein Propionsäurederivat der Formel VII

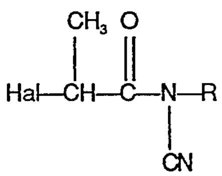 (VII)

worin R die unter Formel I gegebene Bedeutung hat und Hal für Chlor, Brom, Tosyl oder Mesyl steht, in Gegenwart eines säurebindenden Mittels mit einem Hydrochinonderivat der Formel VIII

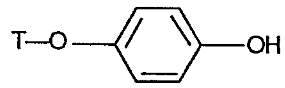 (VIII)

worin T die unter Formel I gegebene Bedeutung hat, umsetzt.

18. Die Verwendung der Wirkstoffe der Formel I, gemäss Anspruch 1, oder sie enthaltender Mittel zur Bekämpfung von Unkräutern.

19. Die Verwendung gemäss Anspruch 18, zur selektiven Unkrautbekämpfung.

20. Die Verwendung gemäss Anspruch 19, zur selektiven Bekämpfung von monokotylen Unkräutern.

21. Die Verwendung gemäss Anspruch 20, zur selektiven pre- oder postemergenten Bekämpfung von monokotylen Unkräutern in Nutzpflanzenkulturen.

22. Die Verwendung gemäss Anspruch 21 in dikotylen Nutzpflanzenkulturen wie Soja, Raps, Zuckerrüben und Baumwolle.

23. Verfahren zur Herstellung der Verbindungen der Formel Ic

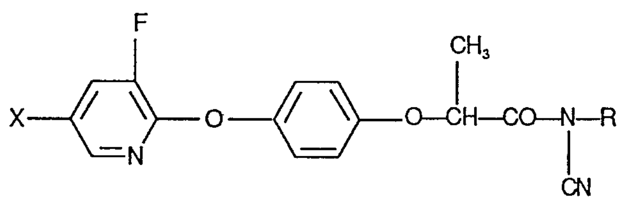 (Ic)

worin R und X die unter Formel I gegebene Bedeutung haben, dadurch gekennzeichnet, dass man entweder

a) ein 3-Nitropyridin der Formel XIII

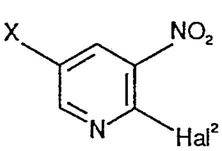 (XIII)

worin X die unter Formel I gegebene Bedeutung hat und $Hal^2$ für Fluor, Chlor oder Brom steht, in Gegenwart einer Base mit Hydrochinon zu einer Verbindung der Formel XIV

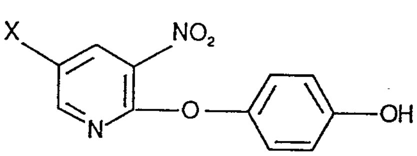 (XIV)

umsetzt, dieses Zwischenprodukt in Gegenwart eines Metallkatalysators zu einer Aminoverbindung der Formel XV

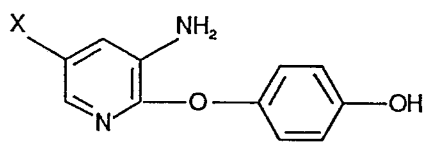 (XV)

reduziert, diese diazotiert und mit einem Fluorierungsmittel in eine Verbindung der Formel XVI

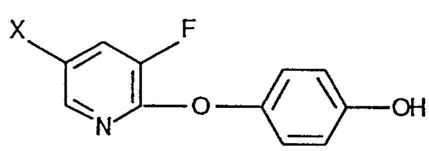 (XVI)

überführt und dieses fluorierte Zwischenprodukt in Gegenwart einer Base mit einem Propionsäure-Derivat der Formel VII umsetzt, oder dass man

b) das Nitropyridin der Formel (XIII) in Gegenwart

einer Base mit einem Propionsäureester der Formel XVII

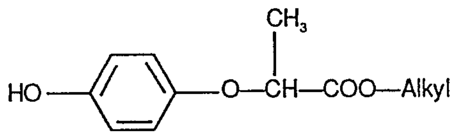
(XVII)

worin Alkyl für $C_1$-$C_4$-Alkyl steht, umsetzt, das erhaltene Zwischenprodukt der Formel XVIII

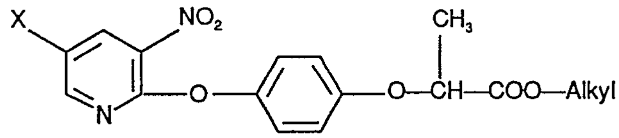
(XVIII)

in Gegenwart eines Metallkatalysators zur Aminoverbindung der Formel XIX

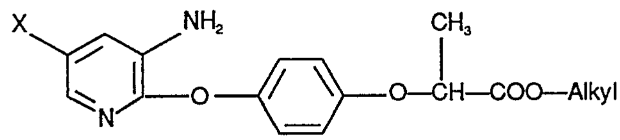
(XIX)

reduziert, dieses Zwischenprodukt diazotiert und mit einem Fluorierungsmittel in eine Verbindung der Formel XX

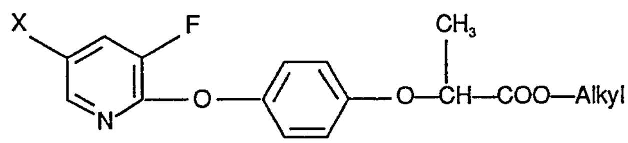
(XX)

überführt, diesen Ester verseift und mit einem Halogenierungsmittel in ein 2-Phenoxypropionsäurehalogenid der Unterformel IIa

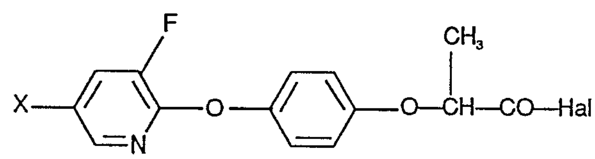
(IIa)

worin Hal und X die unter Formel II gegebene Bedeutung haben, überführt und in Gegenwart einer Base mit einem Cyanamin der Formel III umsetzt.

**Claims for the Contracting States BE, CH, DE, FR, GB, IT, LI, NL, SE**

1. 2-Phenoxypropionic acid cyanamides of formula I

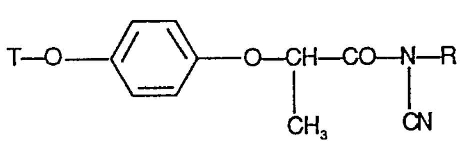
(I)

wherein R is hydrogen, $C_1$-$C_4$alkyl, $C_3$-$C_4$alkenyl, $C_3$-$C_4$alkynyl or $C_2$-$C_4$alkoxyalkyl and T is a radical

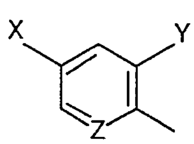
,

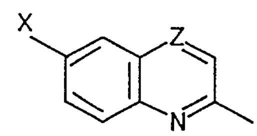
or
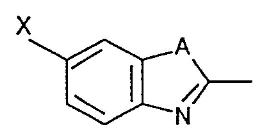

wherein A is oxygen or sulfur, X is fluorine, chlorine, bromine, iodine or trifluoromethyl, Y is hydrogen, fluorine, chlorine, bromine or trifluoromethyl, and Z is nitrogen or a methine bridge.

2. Compounds according to claim 1 wherein R is hydrogen or $C_1$-$C_4$alkyl.

3. Compounds according to claim 2 wherein R is $C_1$-$C_4$alkyl.

4. Compounds according to claim 1 wherein T is the radical

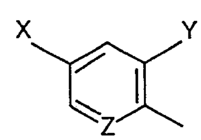

wherein X, Y and Z are as defined in claim 1.

5. Compounds according to claim 1 wherein T is the radical

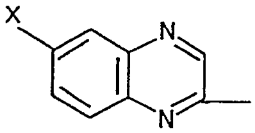

wherein X is as defined in claim 1.

6. A compound according to claim 5 wherein X is fluorine or chlorine.

7. Compounds according to claim 1 wherein T is the radical

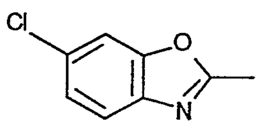

8. Compounds according to claim 4 wherein X is chlorine, Y is fluorine and Z is nitrogen.

9. Compounds according to claim 1 wherein R is $C_1$–$C_4$alkyl and T is 6-fluoroquinoxalin-2-yl.

10. Compounds according to claim 1 wherein R is $C_1$–$C_4$alkyl and T is 6-chloroquinoxalin-2-yl.

11. Compounds according to claim 1 wherein R is $C_1$–$C_4$alkyl and T is 6-chlorobenzoxazol-2-yl.

12. Compounds according to claim 1 wherein R is $C_1$–$C_4$alkyl and T is 5-chloro-3-fluoropyridin-2-yl.

13. 2-[4-(5-chloro-3-fluoropyridin-2-yloxy)-phenoxy]-propionic acid N-cyano-N-methylamide according to claim 1.

14. 2-[4-(5-trifluoromethylpyridin-2-yloxy)-phenoxy]-propionic acid N-cyano-N-methylamide according to claim 1.

15. 2-[4-(5-chloro-3-fluoropyridin-2-yloxy)-phenoxy]-propionic acid N-cyano-N-ethylamide according to claim 1.

16. 2-[4-(6-chloroquinoxalin-2-yloxy)-phenoxy]-propionic acid N-cyano-N-ethylamide according to claim 1.

17. 2-[4-(6-chloroquinoxalin-2-yloxy)-phenoxy]-propionic acid N-cyano-N-methylamide according to claim 1.

18. 2-[4-(6-fluoroquinoxalin-2-yloxy)-phenoxy]-propionic acid N-cyano-N-ethylamide according to claim 1.

19. 2-[4-(6-fluoroquinoxalin-2-yloxy)-phenoxy]-propionic acid N-cyano-N-methylamide according to claim 1.

20. 2-[4-(6-chlorobenzoxazol-2-yloxy)-phenoxy]-propionic acid N-cyano-N-n-butylamide according to claim 1.

21. A process for the preparation of compounds of formula I according to claim 1, which comprises reacting a 2-phenoxy-propionic acid halide of formula II

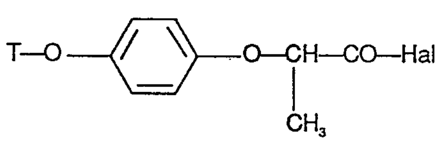 (II),

wherein T is as defined for formula I and Hal is chlorine or bromine, with a cyanamine of formula III

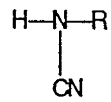 (III),

wherein R is as defined for formula I in claim 1, in the presence of an acid-binding agent.

22. A process for the preparation of compounds of formula Ia

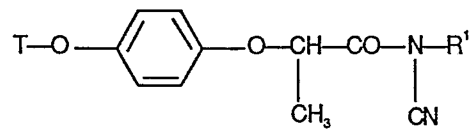 (Ia),

wherein T is as defined for formula I in claim 1 and $R^1$ is $C_1$–$C_4$alkyl, $C_3$–$C_4$alkenyl, $C_3$–$C_4$alkoxyalkyl, which comprises reacting a corresponding cyanamino compound of sub-formula Ib

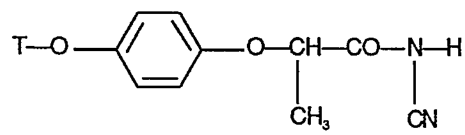 (Ib),

wherein T is as defined for formula I in claim 1, with a halogen compound of formula IV

Hal–$R^1$ (IV),

wherein $R^1$ is as defined for formula Ia und Hal is chlorine, bromine or iodine, in the presence of an acid-binding agent.

23. A process for the preparation of compounds of formula I which comprises reacting a phenoxypropionic acid derivative of formula V

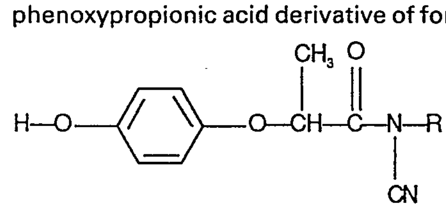 (V)

wherein R is as defined for formula I, with a halide of formula VI

T–Hal (VI),

wherein T is as defined for formula I and Hal is fluorine, chlorine, bromine or iodine, in the presence of an acid-binding agent.

24. A process for the preparation of compounds of formula I which comprises reacting a propionic acid derivative of formula VII

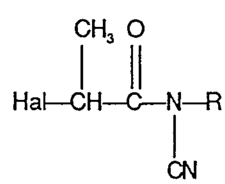 (VII)

wherein R is as defined for formula I and Hal is chlorine, bromine, tosyl or mesyl, with a hydroquinone derivative of formula VIII

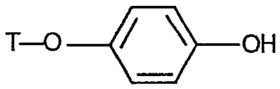

(VIII)

wherein T is as defined for formula I, in the presence of an acid-binding agent.

25. A herbicidal composition which contains as active ingredient at least one 2-phenoxypropionic acid amide of formula I according to claim 1 together with carriers and/or other adjuvants.

26. The use of active ingredients of formula I according to claim 1, or compositions containing them, for controlling weeds.

27. The use according to claim 26 for selective weed control.

28. The use according to claim 27 for the selective control of monocotyledonous weeds.

29. The use according to claim 28 for the selective pre- or post-emergence control of monocotyledonous weeds in crops of useful plants.

30. The use according to claim 29 in crops of useful plants that are dicotyledonous, such as soybeans, rape, sugar beet and cotton.

31. Compounds of formula VII

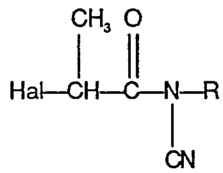

(VII)

wherein R is as defined for formula I in claim 1 and Hal is chlorine, bromine, tosyl or mesyl.

32. Compounds of formula V

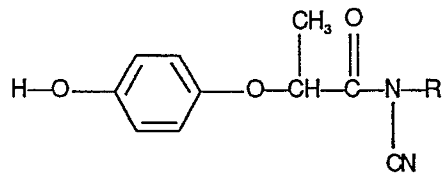

(V)

wherein R is as defined for formula I in claim 1.

33. Compounds of formula XII

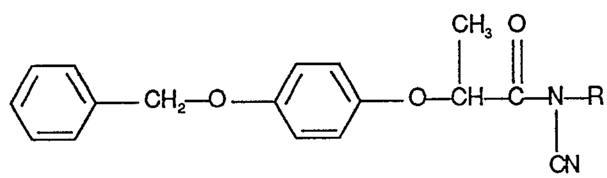

(XII)

wherein R is as defined for formula I in claim 1.

34. A process for the preparation of compounds of formula Ic

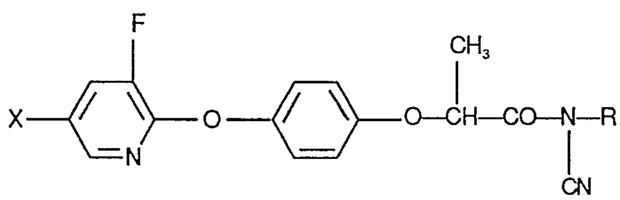

(Ic)

wherein R and X are as defined for formula I, which process comprises either

a) reacting a 3-nitropyridine of formula XIII

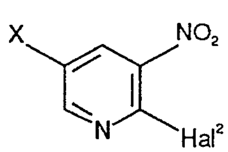

(XIII)

wherein X is as defined for formula I and $Hal^2$ is fluorine, chlorine or bromine, with hydroquinone, in the presence of a base, to give a compound of formula XIV

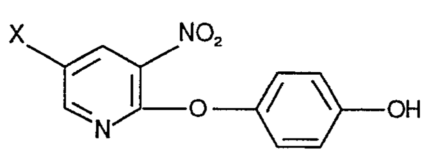

(XIV)

reducing this intermediate in the presence of a metal catalyst to give an amino compound of formula XV

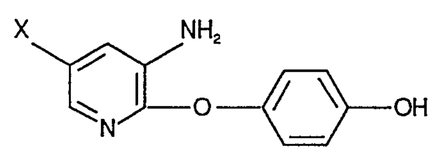

(XV)

diazotising this amino compound and converting it with a fluorinating agent into a compound of formula XVI

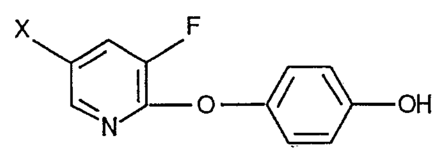

(XVI)

and reacting this fluorinated intermediate with a propionic acid derivative of formula VII in the presence of a base, or

b) reacting the nitropyridine of formula (XIII) with a propionic acid ester of formula XVII

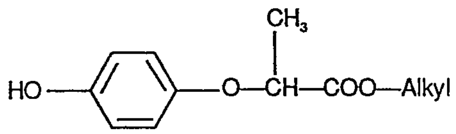

(XVII)

wherein alkyl is $C_1$–$C_4$alkyl, in the presence of a base, reducing the resulting intermediate of formula XVIII

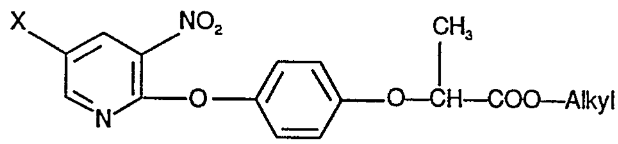

(XVIII)

in the presence of a metal catalyst to give the amino compound of formula XIX

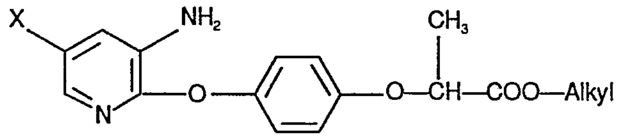

(XIX)

diazotising this intermediate and converting it with a fluorinating agent into a compound of formula XX

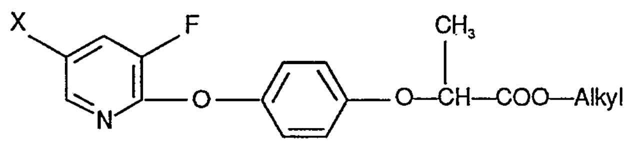

(XX)

hydrolysing this ester and converting it with a halogenating agent into a 2-phenoxypropionic acid halide of sub-formula IIa

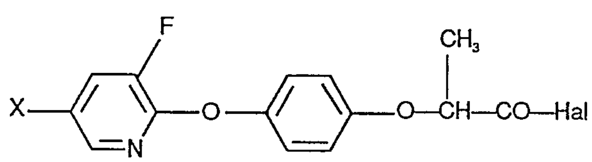

(IIa)

wherein Hal and X are as defined for formula II, and reacting this halide with a cyanamine of formula III in the presence of a base.

**Claims for the Contracting State: AT**

1. A herbicidal composition that contains, together with carriers and/or other adjuvants, at least one 2-phenoxypropionic acid cyanamide of formula I

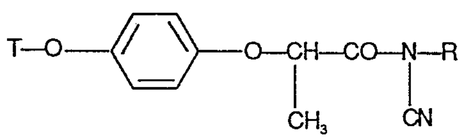

(I)

wherein R is hydrogen, $C_1$–$C_4$alkyl, $C_3$–$C_4$alkenyl, $C_3$–$C_4$alkynyl or $C_2$–$C_4$alkoxyalkyl and T is a radical

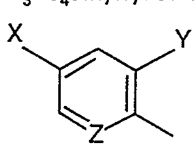

,

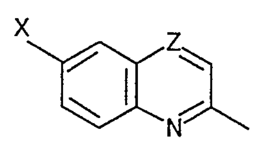

or

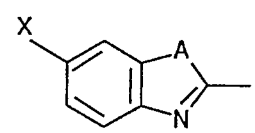

wherein A is oxygen or sulfur, X is fluorine, chlorine, bromine, iodine or trifluoromethyl, Y is hydrogen, fluorine, chlorine, bromine or trifluoromethyl, and Z is nitrogen or a methine bridge.

2. A composition according to claim 1 wherein R is hydrogen or $C_1$–$C_4$alkyl.

3. A composition according to claim 2 wherein R is $C_1$–$C_4$alkyl.

4. A composition according to claim 1 wherein T is the radical

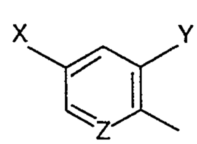

wherein X, Y and Z are as defined in claim 1.

5. A composition according to claim 1 wherein T is the radical

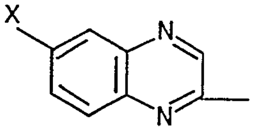

wherein X is as defined in claim 1.

6. A composition according to claim 5 wherein X is fluorine or chlorine.

7. A composition according to claim 1 wherein T is the radical

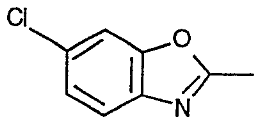

8. A composition according to claim 4 wherein X is chlorine, Y is fluorine and Z is nitrogen.

9. A composition according to claim 1 wherein R is $C_1$–$C_4$alkyl and T is 6-fluoroquinoxalin-2-yl.

10. A composition according to claim 1 wherein R is $C_1$–$C_4$alkyl and T is 6-chloroquinoxalin-2-yl.

11. A composition according to claim 1 wherein R is $C_1$–$C_4$alkyl and T is 6-chlorobenzoxazol-2-yl.

12. A composition according to claim 1 wherein R is $C_1$–$C_4$alkyl and T is 5-chloro-3-fluoropyridin-2-yl.

13. A composition according to claim 1 that contains an active ingredient selected from the group consisting of 2-[4-(5-chloro-3-fluoropyridin-2-yloxy)-phenoxy]-propionic acid N-cyano-N-methylamide, 2-[4-(5-trifluoromethylpyridin-2-yloxy)-phenoxy]-propionic acid N-cyano-N-methylamide, 2-[4-(5-chloro-3-fluoropyridin-2-yloxy)-phenoxy]-propionic acid N-cyano-N-ethylamide, 2-[4-(6-chloroquinoxalin-2-yloxy)-phenoxy]-propionic acid N-cyano-N-ethylamide, 2-[4-(6-chloroquinoxalin-2-yloxy)-phenoxy]-propionic acid N-cyano-N-methylamide, 2-[4-(6-fluoroquinoxalin-2-yloxy)-phenoxy]-propionic acid N-cyano-N-ethylamide, 2-[4-(6-fluoroquinoxalin-2-yloxy)-phenoxy]-propionic acid N-cyano-N-methylamide and 2-[4-(6-chlorobenzoxazol-2-yloxy)-phenoxy]-propionic acid N-cyano-N-n-butylamide.

14. A process for the preparation of compounds of formula I according to claim 1, which comprises reacting a 2-phenoxypropionic acid halide of formula II

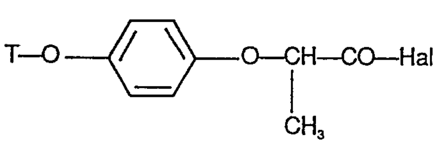 (II),

wherein T is as defined for formula I and Hal is chlorine or bromine, with a cyanamine of formula III

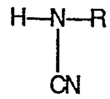 (III),

wherein R is as defined for formula I in claim 1, in the presence of an acid-binding agent.

15. A process for the preparation of compounds of formula Ia

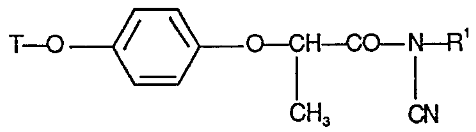 (Ia),

wherein T is as defined for formula I in claim I and $R^1$ is $C_1$–$C_4$alkyl, $C_3$–$C_4$alkenyl, $C_3$–$C_4$alkynyl or $C_2$–$C_4$alkoxyalkyl which comprises reacting a corresponding cyanamino compound of sub-formula Ib

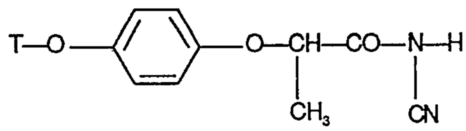 (Ib),

wherein T is as defined for formula I in claim 1, with a halogen compound of formula IV

Hal-$R^1$ (IV),

wherein $R^1$ is as defined for formula Ia and Hal is chlorine, bromine or iodine, in the presence of an acid-binding agent.

16. A process for the preparation of compounds of formula I which comprises reacting a phenoxypropionic acid derivative of formula V

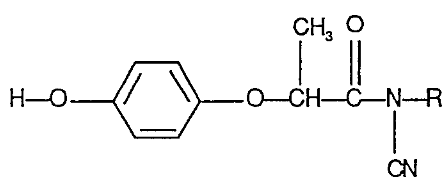 (V)

wherein R is as defined for formula I, with a halide of formula VI

T-Hal (VI),

wherein T is as defined for formula I and Hal is fluorine, chlorine, bromine, or iodine, in the presence of an acid-binding agent.

17. A process for the preparation of compounds of formula I which comprises reacting a propionic acid derivative of formula VII

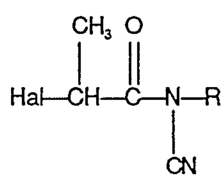 (VII)

wherein R is as defined for formula I and Hal is chlorine, bromine, tosyl or mesyl, with a hydroquinone derivative of formula VIII

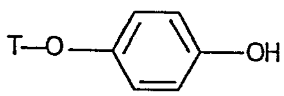

(VIII)

wherein T is as defined for formula I, in the presence of an acid-binding agent.

18. The use of active ingredients of formula I according to claim 1, or compositions containing them, for controlling weeds.

19. The use according to claim 18 for selective weed control.

20. The use according to claim 19 for the selective control of monocotyledonous weeds.

21. The use according to claim 20 for the selective pre- or post-emergence control of monocotyledonous weeds in crops of useful plants.

22. The use according to claim 21 in crops of useful plants that are dicotyledonous, such as soybeans, rape, sugar beet and cotton.

23. A process for the preparation of compounds of formula Ic

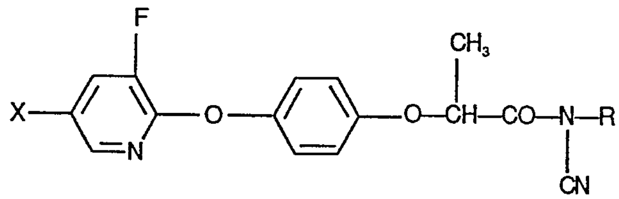

(Ic)

wherein R and X are as defined for formula I, which process comprises either

a) reacting a 3-nitropyridine of formula XIII

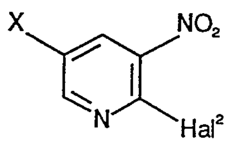

(XIII)

wherein X is as defined for formula I and $Hal^2$ is fluorine, chlorine or bromine, with hydroquinone, in the presence of a base, to give a compound of formula XIV

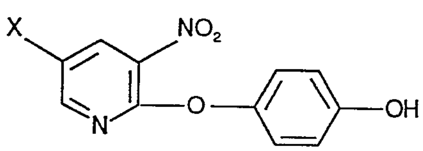

(XIV)

reducing this intermediate in the presence of a metal catalyst to give an amino compound of formula XV

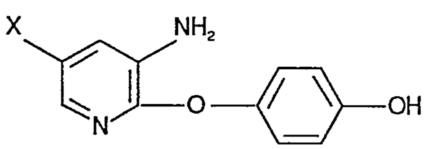

(XV)

diazotising this amino compound and converting it with a fluorinating agent into a compound of formula XVI

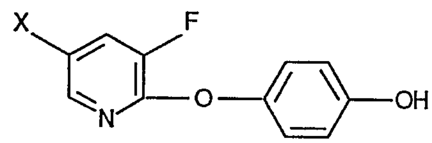

(XVI)

and reacting this fluorinated intermediate with a propionic acid derivative of formula VII in the presence of a base, or

b) reacting the nitropyridine of formula (XIII) with a propionic acid ester of formula XVII

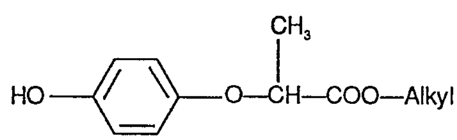

(XVII)

wherein alkyl is $C_1$-$C_4$alkyl, in the presence of a base, reducing the resulting intermediate of formula XVIII

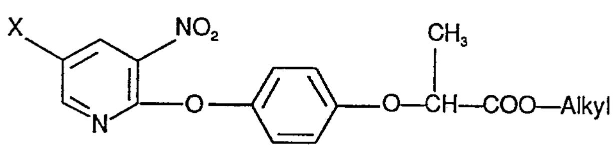

(XVIII)

in the presence of a metal catalyst to give the amino compound of formula XIX

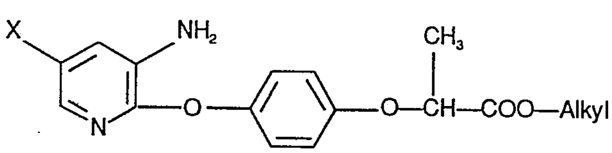

(XIX)

diazotising this intermediate and converting it with a fluorinating agent into a compound of formula XX

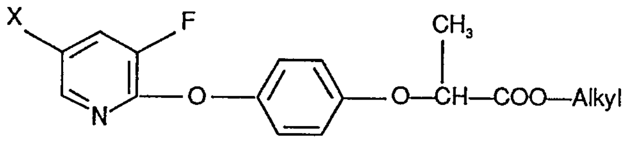

(XX)

hydrolysing this ester and converting it with a halogenating agent into a 2-phenoxypropionic acid halide of sub-formula IIa

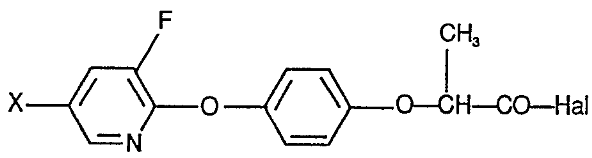

(IIa)

wherein Hal and X are as defined for formula II, and reacting this halide with a cyanamine of formula III in the presence of a base.

**Revendications pour les Etats contractants: BE, CH, DE, FR, GB, IT, LI, NL, SE**

1. Cyanamides d'acides 2-phénoxypropioniques de formule I

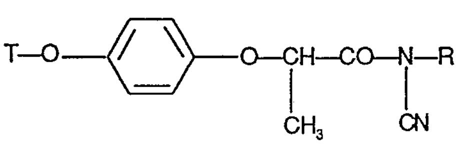

(I)

dans laquelle
R représente l'hydrogène, un groupe alkyle en $C_1$-$C_4$, alcényle en $C_3$-$C_4$, alcynyle en $C_3$-$C_4$ ou alcoxyalkyle en $C_2$-$C_4$, et
T représente un groupe

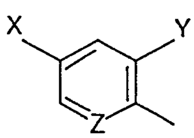

,

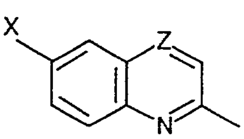

ou

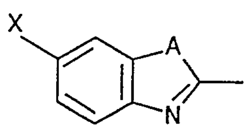

dans lequel
A représente l'oxygène ou le soufre,
X représente le fluor, le chlore, le brome, l'iode ou un groupe trifluorométhyle,
Y représente l'hydrogène, le fluor, le chlore, le brome ou un groupe trifluorométhyle, et
Z représente l'azote ou le pont méthine.

2. Composés selon la revendication 1, caractérisés en ce que R représente l'hydrogène ou un groupe alkyle en $C_1$-$C_4$.

3. Composés selon la revendication 2, caractérisés en ce que R représente un groupe alkyle en $C_1$-$C_4$.

4. Composés selon la revendication 1, caractérisés en ce que T représente le groupe

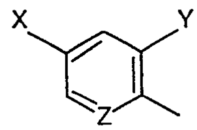

dans lequel X, Y et Z ont les significations indiquées dans la revendication 1.

5. Composés selon la revendication 1, caractérisés en ce que T représente le groupe

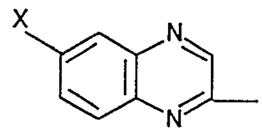

dans lequel X a les significations indiquées dans la revendication 1.

6. Composé selon la revendication 5, caractérisé en ce que X représente le fluor ou le chlore.

7. Composés selon la revendication 1, caractérisés en ce que T représente le groupe

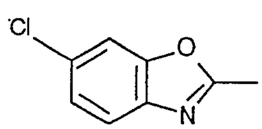

8. Composés selon la revendication 4, caractérisés en ce que X représente le chlore, Y le fluor et Z l'azote.

9. Composés selon la revendication 1, caractérisés en ce que R représente un groupe alkyle en $C_1$-$C_4$ et T le groupe 6-fluoro-quinoxaline-2-yle.

10. Composés selon le revendication 1, caractérisés en ce que R représente un groupe alkyle en $C_1$-$C_4$ et T le groupe 6-chloro-quinoxaline-2-yle.

11. Composés selon la revendication 1, caractérisés en ce que R représente un groupe alkyle an $C_1$-$C_4$ et T le groupe 6-chloro-benzoxazole-2-yle.

12. Composés selon la revendication 1, caractérisés en ce que R représente un groupe alkyle en $C_1$-$C_4$ et T le groupe 5-chloro-3-fluoropyridine-2-yle.

13. Le N-cyano-N-méthyl-amide de l'acide 2-[4-(5-chloro-3-fluoropyridine-2-yloxy)-phénoxy]-propionique selon la revendication 1.

14. Le N-cyano-N-méthyl-amide de l'acide 2-[4-(5-trifluorométhylpyridine-2-yloxy)-phénoxy]-propionique selon la revendication 1.

15. Le N-cyano-N-éthyl-amide de l'acide 2-[4-(5-chloro-3-fluoropyridine-2-yloxy)-phénoxy]-propionique selon la revendication 1.

16. Le N-cyano-N-éthyl-amide de l'acide 2-[4-(6-chloro-quinoxaline-2-yloxy)-phé noxy]-propionique selon la revendication 1.

17. Le N-cyano-N-méthyl-amide de l'acide 2-[4-(6-chloro-quinoxaline-2-yloxy)-phé noxy]-propionique selon la revendication 1.

18. Le N-cyano-N-éthyl-amide de l'acide 2-[4-(6-fluoro-quinoxaline-2-yloxy)-phé noxy]-phénoxy]-propionique selon la revendication 1.

19. Le N-cyano-N-méthyl-amide de l'acide 2-[4-(6-fluoro-quinoxaline-2-yloxy)-phé noxy]-propionique selon la revendication 1.

20. Le N-cyano-N-n-butyl-amide de l'acide 2-[4-(6-chloro-benzoxazole-2-yloxy)-phé noxy]-propionique selon la revendication 1.

21. Procédé de préparation des composés de formule I selon la revendication 1, caractérisé en ce que l'on fait réagir un halogénure d'acide 2-phénoxy-propionique de formule II

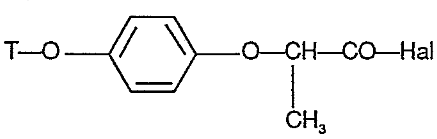

(II),

dans laquelle T a les significations indiquées en référence à la formule I et Hal représente le chlore ou le brome, en présence d'un capteur d'acide, avec une cyanamine de formule III

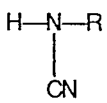

(III),

dans laquelle R a les significations indiquées en référence à la formule I dans la revendication 1.

22. Procédé de préparation des composés de formule Ia

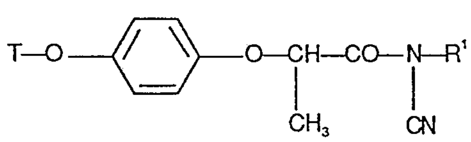

(Ia),

dans laquelle T a les significations indiquées en référence à la formule I dans la revendication 1 et $R^1$ représente un groupe alkyle en $C_1$-$C_4$, alcényle en $C_3$-$C_4$, alcynyle en $C_3$-$C_4$ ou alcoxyalkyle en $C_2$-$C_4$, caractérisé en ce que l'on fait réagir un composé cyanaminé correspondant de formule partielle Ib

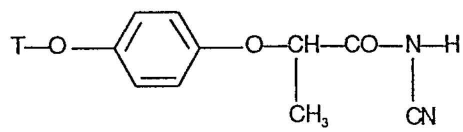

(Ib),

dans laquelle T a les significations indiquées en référence à la formule I dans la revendication 1, en présence d'un capteur d'acide, avec un dérivé halogéné de formule IV

$Hal\text{-}R^1$ (IV),

dans laquelle $R^1$ a les significations indiquées en référence à la formule Ia et Hal représente le chlore, le brome ou l'iode.

23. Procédé de préparation des composés de formule I, caractérisé en ce que l'on fait réagir un dérivé d'acide phénoxypropionique de formule V

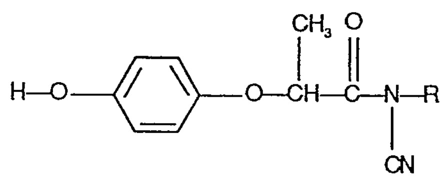

(V)

dans laquelle R a les significations indiquées en référence à la formule I, en présence d'un capteur d'acide, avec un halogénure de formule VI

T-Hal (VI),

dans laquelle T a les significations indiquées en référence à la formule I et Hal représente le fluor, le chlore, le brome ou l'iode.

24. Procédé de préparation des composés de formule I, caractérisé en ce que l'on fait réagir un dérivé d'acide propionique de formule VII

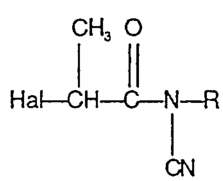

(VII)

dans laquelle R a les significations indiquées en référence à la formule I et Hal représente le chlore, le brome, un groupe toluène-sulfonyle ou méthane-sulfonyle, en présence d'un capteur d'acide, avec un dérivé de l'hydroquinone répondant à la formule VIII

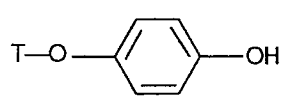

(VIII)

dans laquelle T a les significations indiquées en référence à la formule I.

25. Un produit herbicide, caractérisé en ce qu'il contient en tant que substance active, avec des véhicules et/ou d'autres additifs, au moins un amide d'acide 2-phénoxypropionique de formule I selon la revendication 1.

26. L'utilisation des substances actives de formule I selon la revendication 1 ou de produits en contenant dans la lutte contre les mauvaises herbes.

27. L'utilisation selon la revendication 26, dans la lutte sélective contre les mauvaises herbes.

28. L'utilisation selon la revendication 27, pour la lutte sélective contre les mauvaises herbes monocotylédones.

29. L'utilisation selon la revendication 28, pour la lutte sélective en pré- ou post-levée contre les mauvaises herbes monocotylédones dans les cultures de végétaux utiles.

30. L'utilisation selon la revendication 29, dans les cultures de végétaux utiles dicotylédones tels que le soja, le colza, la betterave à sucre et le coton.

31. Composés de formule VII

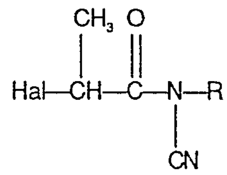

(VII)

dans laquelle R a les significations indiquées en référence à la formule I dans la revendication 1 et Hal représente le chlore, le brome, un groupe toluènesulfonyle ou méthane-sulfonyle.

32. Composés de formule V

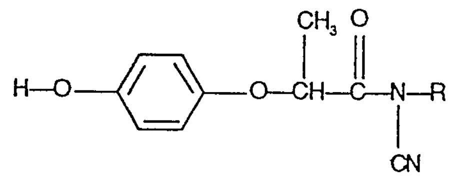

(V)

dans laquelle R a les significations indiquées en référence à la formule I dans la revendication 1.

33. Composés de formule XII

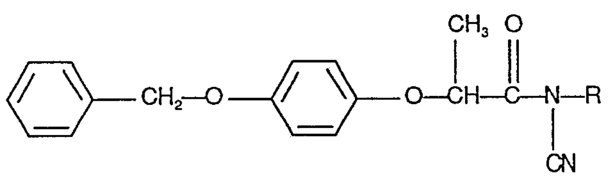

(XII)

dans laquelle R a les significations indiquées en référence à la formule I dans la revendication 1.

34. Procédé de préparation des composés de formule Ic

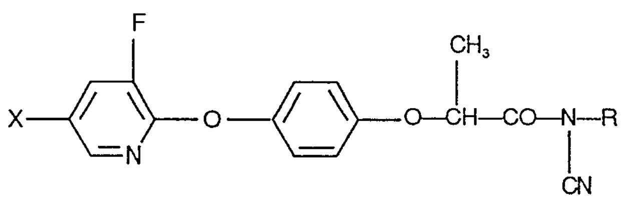

(Ic)

dans laquelle R et X ont les significations indiquées en référence à la formule I, caractérisé en ce que:

a) ou bien on fait réagir une 3-nitropyridine de formule XIII

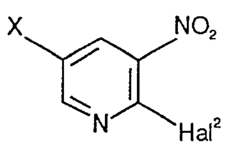

(XIII)

dans laquelle X a les significations indiquées en référence à la formule I et $Hal^2$ représente le fluor, le chlore ou le brome, en présence d'une base, avec l'hydroquinone, ce qui donne un composé de formule XIV

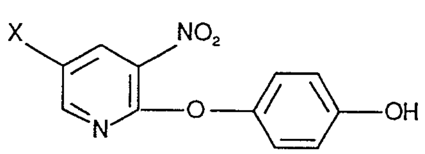

(XIV)

produit intermédiaire qu'on réduit, en présence d'un catalyseur métallique, en un composé aminé de formule XV

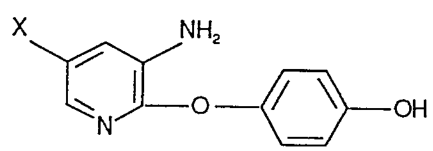

(XV)

qu'on diazote et qu'on convertit à l'aide d'un agent fluorant en un composé de formule XVI

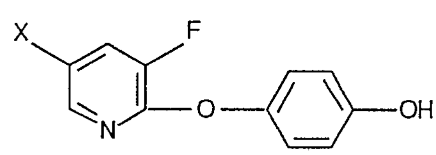

(XVI)

après quoi on fait réagir ce produit intermédiaire fluoré, en présence d'une base, avec un dérivé d'acide propionique de formule VII, ou bien

b) on fait réagir la nitropyridine de formule (XIII) en présence d'une base avec un ester propionique de formule XVII

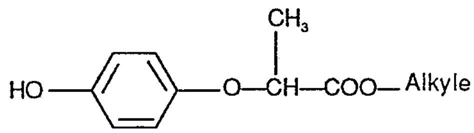

(XVII)

dans laquelle «alkyle» représente un groupe alkyle en $C_1$-$C_4$, ce qui donne un produit intermédiaire de formule XVIII

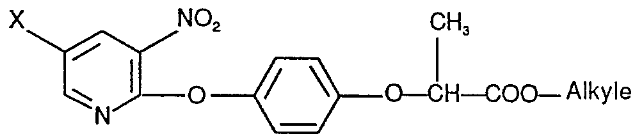

(XVIII)

qu'on réduit en présence d'un catalyseur métallique en le composé aminé de formule XIX

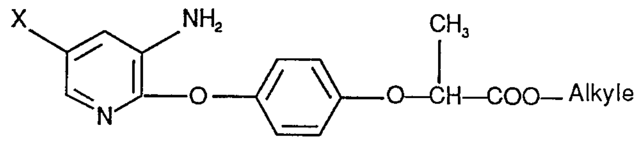

(XIX)

on diazote ce produit intermédiaire et on le convertit à l'aide d'un agent fluorant en un composé de formule XX

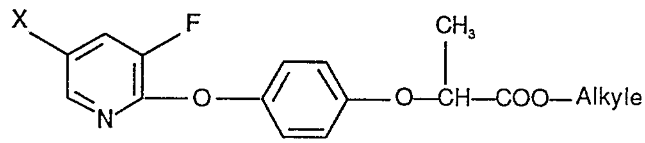

(XX)

on saponifie cet ester et on convertit à l'aide d'un agent halogénant en un halogénure d'acide 2-phénoxypropionique de formule partielle IIa

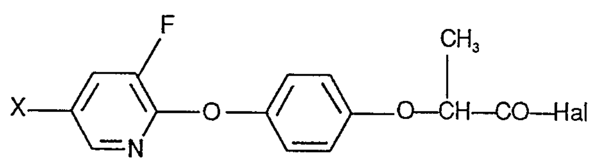

(IIa)

dans laquelle Hal et X ont les significations indiquées en référence à la formule II, et on fait réagir en présence d'une base avec une cyanamine de formule III.

**Revendications pour l'Etat contractant: AT**

1. Un produit herbicide, caractérisé en ce qu'il contient, avec des véhicules et/ou d'autres additifs, au moins un cyanamide d'acide 2-phénoxypropionique de formule I

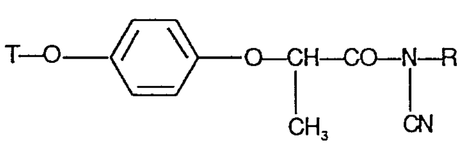

(I)

dans laquelle
R représente l'hydrogène, un groupe alkyle en $C_1$-$C_4$, alcényle en $C_3$-$C_4$, alcynyle en $C_3$-$C_4$ ou alcoxyalkyle en $C_2$-$C_4$, et

T représente un groupe

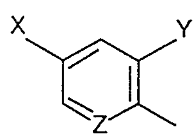

,

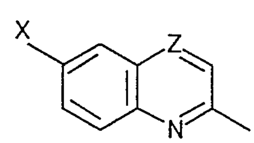

ou

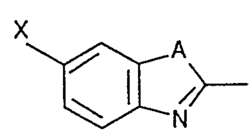

dans lequel
A représente l'oxygène ou le soufre,
X représente le fluor, le chlore, le brome, l'iode ou un groupe trifluorométhyle,
Y représente l'hydrogène, le fluor, le chlore, le brome ou un groupe trifluorométhyle, et
Z représente l'azote ou le pont méthine.

2. Produit selon la revendication 1, caractérisé

en ce que R représente l'hydrogène ou un groupe alkyle en $C_1$–$C_4$.

3. Produit selon la revendication 2, caractérisé en ce que R représente un groupe alkyle en $C_1$-$C_4$.

4. Produit selon la revendication 1, caractérisé en ce que T représente le groupe

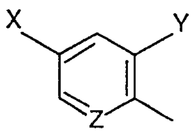

dans lequel X, Y et Z ont les significations indiquées dans la revendication 1.

5. Produit selon la revendication 1, caractérisé en ce que T représente le groupe

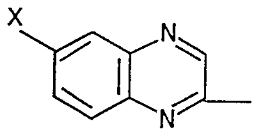

dans lequel X a les significations indiquées dans la revendication 1.

6. Produit selon la revendication 5, caractérisé en ce que X représente le fluor ou le chlore.

7. Produit selon la revendication 1, caractérisé en ce que T représente le groupe

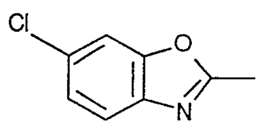

8. Produit selon la revendication 4, caractérisé en ce que X représente le chlore, Y le fluor et Z l'azote.

9. Produit selon la revendication 1, caractérisé en ce que R représente un groupe alkyle en $C_1$–$C_4$ et T le groupe 6-fluoro-quinoxaline-2-yle.

10. Produit selon la revendication 1, caractérisé en ce que R représente un groupe alkyle en $C_1$–$C_4$ et T le groupe 6-chloro-quinoxaline-2-yle.

11. Produit selon le revendication 1, caractérisé en ce que R représente un groupe alkyle en $C_1$–$C_4$ et T le groupe 6-chloro-benzoxazole-2-yle.

12. Produit selon la revendication 1, caractérisé en ce que R représente un groupe alkyle en $C_1$–$C_4$ et T le groupe 5-chloro-3-fluoropyridine-2-yle.

13. Produit selon la revendication 1, caractérisé en ce qu'il contient une substance active du groupe des suivantes: N-cyano-N-méthyl-amide de l'acide 2-[4-(5-chloro-3-fluoropyridine-2-yloxy)-phénoxy]-propionique, N-cyano-N-méthyl-amide de l'acide 2-[4-(5-trifluorométhyl-pyridine-2-yloxy)-phénoxy]-propionique, N-cyano-N-éthyl-amide de l'acide 2-[4-(5-chloro-3-fluoropyridine-2-yloxy)-phénoxy]-propionique, N-cyano-N-éthyl-amide de l'acide 2-[4-(6-chloro-quinoxaline-2-yloxy)-phénoxy]-propionique, N-cyano-N-méthyl-amide de l'acide 2-[4-(6-chloro-quinoxaline-2-yloxy)-phénoxy]-propionique, N-cyano-N-éthyl-amide de l'acide 2-[4-(6-fluoro-quinoxaline-2-yloxy)-phénoxy]-propionique, N-cyano-N-méthyl-amide de l'acide 2-[4-(6-fluoro-quinoxaline-2-yloxy)-phénoxy]-propionique, et N-cyano-N-n-butyl-amide de l'acide 2-[4-(6-chlorobenzoxazole-2-yloxy)-phénoxy]-propionique.

14. Procédé de préparation des composés de formule I selon la revendication 1, caractérisé en ce que l'on fait réagir un halogénure d'acide 2-phénoxy-propionique de formule II

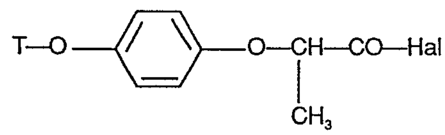 (II) ,

dans laquelle T a les significations indiquées en référence à la formule I et Hal représente le chlore ou le brome, en présence d'un capteur d'acide, avec une cyanamine de formule III

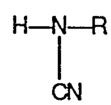 (III) ,

dans laquelle R a les significations indiquées en référence à la formule I dans la revendication 1.

15. Procédé de préparation des composés de formule Ia

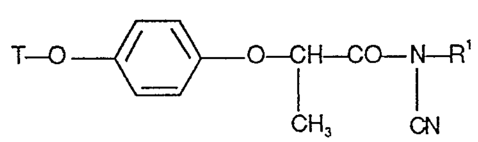 (Ia) ,

dans laquelle T a les significations indiquées en référence à la formule I dans la revendication 1 et $R^1$ représente un groupe alkyle en $C_1$–$C_4$, alcényle en $C_3$–$C_4$, alcynyle en $C_3$–$C_4$ ou alcoxyalkyle en $C_2$–$C_4$, caractérisé en ce que l'on fait réagir un composé cyanaminé correspondant de formule partielle Ib.

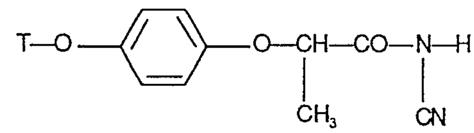 (Ib) ,

dans laquelle T a les significations indiquées en référence à la formule I dans la revendication 1, en présence d'un capteur d'acide, avec un dérivé halogéné de formule IV

$Hal\text{-}R^1$ (IV)

dans laquelle $R^1$ a les significations indiquées en référence à la formule Ia et Hal représente le chlore, le brome ou l'iode.

16. Procédé de préparation des composés de formule I, caractérisé en ce que l'on réagir un dérivé d'acide phénoxypropionique de formule V

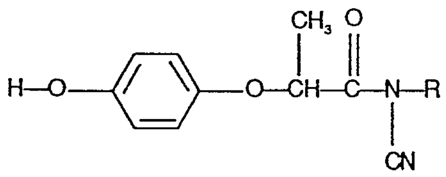

(V)

dans laquelle R a les significations indiquées en référence à la formule I, en présence d'un capteur d'acide, avec un halogénure de formule VI

T-Hal (VI)

dans laquelle T a les significations indiquées en référence à la formule I et Hal représente le fluor, le chlore, le brome ou l'iode.

17. Procédé de préparation des composés de formule I, caractérisé en ce que l'on fait réagir un dérivé d'acide propionique de formule VII

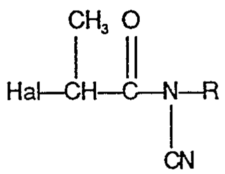

(VII)

dans laquelle R a les significations indiquées en référence à la formule I et Hal représente le chlore, le brome, un groupe toluène-sulfonyle ou méthane-sulfonyle, en présence d'un capteur d'acide, avec un dérivé de l'hydroquinone de formule VIII

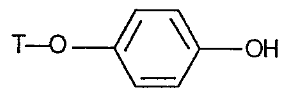

(VIII)

dans laquelle T a les significations indiquées en référence à la formule I.

18. L'utilisation des substances actives de formule I selon la revendication 1 ou de produits en contenant pour la lutte contre les mauvaises herbes.

19. L'utilisation selon la revendication 18, pour la lutte sélective contre les mauvaises herbes.

20. L'utilisation selon la revendication 19, pour la lutte sélective contre les mauvaises herbes monocotylédones.

21. L'utilisation selon la revendication 20, pour la lutte sélective en pré- ou post-levée contre les mauvaises herbes monocotylédones dans les cultures de végétaux utiles.

22. L'utilisation selon la revendication 21, dans des cultures de végétaux utiles dicotylédones tels que le soja, le colza, la betterave à sucre et le coton.

23. Procédé de préparation des composés de formule Ic

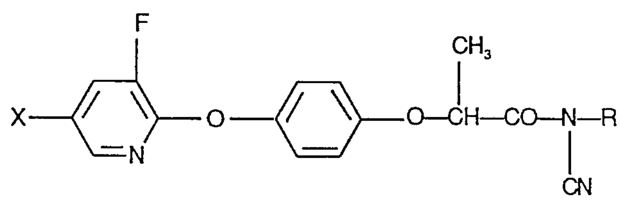

(Ic)

dans laquelle R et X ont les significations indiquées en référence à la formule I caractérisé en ce que:

a) ou bien on fait réagir une 3-nitropyridine de formule XIII

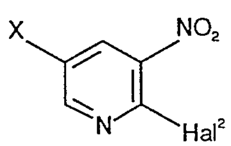

(XIII)

dans laquelle X a les significations indiquées en référence à la formule I et $Hal^2$ représente le fluor, le chlore ou le brome, en présence d'une base, avec l'hydroquinone, ce qui donne un composé de formule XIV

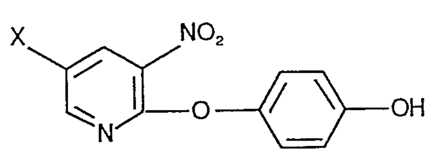

(XIV)

produit intermédiaire qu'on réduit en présence d'un catalyseur métallique en un composé aminé de formule XV

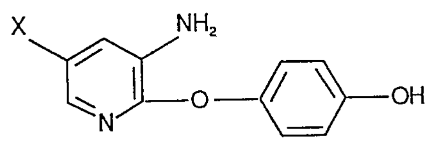

(XV)

qu'on diazote et qu'on convertit à l'aide d'un agent fluorant en un composé de formule XVI

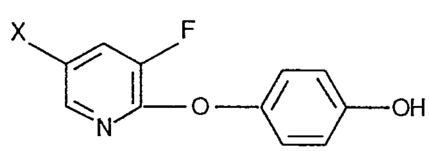

(XVI)

après quoi on fait réagir ce produit intermédiaire fluoré en présence d'une base avec un dérivé d'acide propionique de formule VII, ou bien

b) on fait réagir la nitropyridine de formule XIII en présence d'une base avec un ester propionique de formule XVII

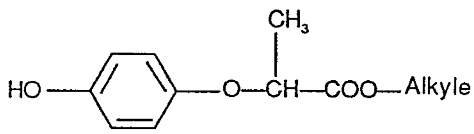

(XVII)

dans laquelle «alkyle» représente un groupe alkyle en $C_1$-$C_4$, ce qui donne le produit intermédiaire de formule XVIII

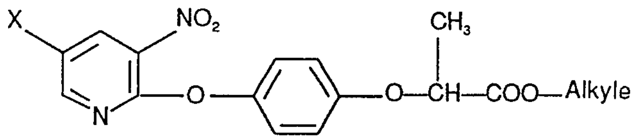

(XVIII)

qu'on réduit en présence d'un catalyseur métallique en le composé aminé de formule XIX

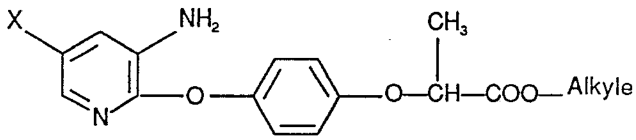

(XIX)

produit intermédiaire qu'on diazote et qu'on convertit à l'aide d'un agent fluorant en un composé de formule XX

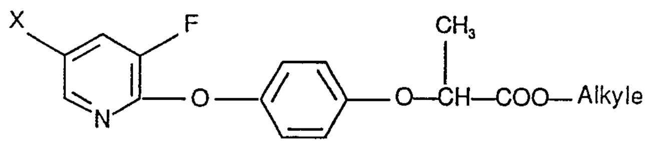

(XX)

on saponifie cet ester et on le convertit à l'aide d'un agent halogénant en un halogénure d'acide phénoxypropionique de formule partielle IIa

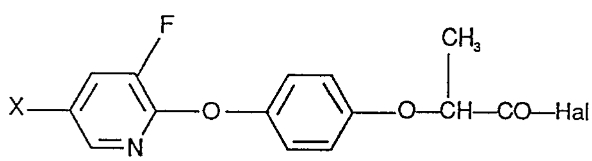

(IIa)

dans laquelle Hal et X ont les significations indiquées en référence à la formule II, et on fait réagir en présence d'une base avec une cyanamine de formule III.